# EUROPÄISCHE PATENTSCHRIFT

(11) **EP 0 580 553 B1**
(45) Veröffentlichungstag und Bekanntmachung des Hinweises auf die Patenterteilung: **26.11.2003**
(21) Anmeldenummer: 93810500.4
(22) Anmeldetag: 13.07.1993
(51) Int. Cl.: C07D 413/06, C07D 417/06, C07D 497/04, C07D 498/04, C07D 471/04, C07D 487/04, A01N 43/72, A01N 43/88, A01N 43/90

(54) **Oxadiazinderivate**
Oxadiazine derivatives
Dérivés d'oxadiazine

(30) Priorität: 22.07.1992 CH 231592
(43) Veröffentlichungstag der Anmeldung: 26.01.1994
(73) Patentinhaber: Syngenta Participations AG, 4058 Basel (CH)
(72) Erfinder: Maienfisch, Peter, Dr., CH-4118 Rodersdorf (CH); Gsell, Laurenz, Dr., CH-4056 Basel (CH)

(56) Entgegenhaltungen:
- EP-A- 0 225 854
- EP-A- 0 230 863
- EP-A- 0 235 725
- EP-A- 0 277 317

## Beschreibung

Die Erfindung betrifft Verbindungen der Formel 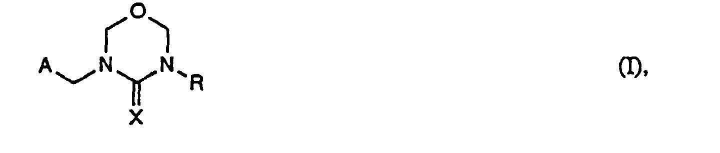 worin
A eine Pyridyl-, 1-Oxidopyridinio- oder Thiazolyl-Gruppe, die unsubstituiert oder ein- oder zweifach durch Substituenten, ausgewählt aus der Gruppe, bestehend aus Halogen, C₂-C₃-Alkenyl, Halogen-C₁-C₃-alkyl, C₁-C₃-Alkyl, und Halogen-C₁-C₃-alkoxy, substituiert ist;
R Wasserstoff, C₁-C₆-Alkyl, Phenyl-C₁-C₄-alkyl, C₃-C₆-Cycloalkyl, C₂-C₆-Alkenyl oder C₂-C₆-Alkinyl; und
X N-NO₂ oder N-CN
bedeuten, in freier Form oder in Salzform, gegebenenfalls Tautomere, in freier Form oder in Salzform, dieser Verbindungen, ein Verfahren zur Herstellung und die Verwendung dieser Verbindungen und Tautomeren, Schädlingsbekämpfungsmittel, deren Wirkstoff aus diesen Verbindungen und Tautomeren, jeweils in freier Form oder in agrochemisch verwendbarer Salzform, ausgewählt ist, ein Verfahren zur Herstellung und die Verwendung dieser Mittel, mit diesen Mitteln behandeltes pflanzliches Vermehrungsgut, ein Verfahren zur Bekämpfung von Schädlingen, Zwischenprodukte, in freier Form oder in Salzform, zur Herstellung dieser Verbindungen, Tautomere, in freier Form oder in Salzform, dieser Zwischenprodukte und ein Verfahren zur Herstellung und die Verwendung dieser Zwischenprodukte und Tautomeren.

In der Literatur, z. B. in EP-A-0 225 854, in EP-A-0 386 565 und in EP-A-0 230 863, werden gewisse Oxadiazinderivate als arthropodazid wirkende Wirkstoffe in Schädlingsbekämpfungsmitteln vorgeschlagen. In EP-A-0 277 317 und in EP-A-0 235 725 werden heterocyclische Verbindungen mit Imino-Substituenten als insektizid wirksame Substanzen beschrieben. Die biologischen Eigenschaften dieser bekannten Verbindungen vermögen auf dem Gebiet der Schädlingsbekämpfung jedoch nicht voll zu befriedigen, weshalb das Bedürfnis besteht, weitere Verbindungen mit schädlingsbekämpfenden Eigenschaften, insbesondere zur Bekämpfung von Insekten, zur Verfügung zu stellen, wobei diese Aufgabe erfindungsgemäss durch die Bereitstellung der vorliegenden Verbindungen I gelöst wird.

Die Verbindungen I können teilweise als Tautomere vorliegen. Bedeutet z. B. R Wasserstoff, können entsprechende Verbindungen I, also solche mit einer 3-H-4-Imino-perhydro-1,3,5-oxadiazin-Teilstrukrtur, im Gleichgewicht mit den jeweiligen Tautomeren vorliegen, die eine 4-Amino-1,2,5,6-tetrahydro-1,3,5-oxadiazin-Teilstruktur aufweisen. Demgemäss sind unter den Verbindungen I vorstehend und nachfolgend gegebenenfalls auch entsprechende Tautomere zu verstehen, auch wenn letztere nicht in jedem Fall speziell erwähnt werden.

Verbindungen I, welche mindestens ein basisches Zentrum aufweisen, können z. B. Säureadditionssalze bilden. Diese werden beispielsweise mit starken anorganischen Säuren, wie Mineralsäuren, z. B. Perchlorsäure, Schwefelsäure, Salpetersäure, salpetrige Säure, einer Phosphorsäure oder einer Halogenwasserstoffsäure, mit starken organischen Carbonsäuren, wie gegebenenfalls, z. B. durch Halogen, substituierten C₁-C₄-Alkancarbonsäuren, z. B. Essigsäure, wie gegebenenfalls ungesättigten Dicarbonsäuren, z. B. Oxal-, Malon-, Bernstein-, Malein-, Fumar- oder Phthalsäure, wie Hydroxycarbonsäuren, z. B. Ascorbin-, Milch-, Äpfel-, Wein- oder Zitronensäure, oder wie Benzoesäure, oder mit organischen Sulfonsäuren, wie gegebenenfalls, z. B. durch Halogen, substituierten C₁-C₄-Alkan- oder Aryl-sulfonsäuren, z. B. Methan- oder p-Toluolsulfonsäure, gebildet. Ferner können Verbindungen I mit mindestens einer aciden Gruppe Salze mit Basen bilden. Geeignete Salze mit Basen sind beispielsweise Metallsalze, wie Alkali- oder Erdalkalimetallsalze, z. B. Natrium-, Kalium- oder Magnesiumsalze, oder Salze mit Ammoniak oder einem organischen Amin, wie Morpholin, Piperidin, Pyrrolidin, einem Mono-, Di- oder Triniederalkylamin, z. B. Ethyl-, Diethyl-, Triethyl- oder Dimethyl-propyl-amin, oder einem Mono-, Dioder Trihydroxyniederalkylamin, z. B. Mono-, Di- oder Triethanolamin. Weiterhin können gegebenenfalls entsprechende innere Salze gebildet werden. Bevorzugt sind im Rahmen der Erfindung agrochemisch vorteilhafte Salze; umfasst sind aber auch für agrochemische Verwendungen mit Nachteilen behaftete, z. B. bienen- oder fisch-toxische, Salze, die beispielsweise für die Isolierung bzw. Reinigung von freien Verbindungen I oder deren agrochemisch verwendbaren Salzen eingesetzt werden. Infolge der engen Beziehung zwischen den Verbindungen I in freier Form und in Form ihrer Salze sind vorstehend und nachfolgend unter den freien Verbindungen I bzw. ihren Salzen sinn- und zweckgemäss gegebenenfalls auch die entsprechenden Salze bzw. die freien Verbindungen I zu verstehen. Entsprechendes gilt für Tautomere von Verbindungen I und deren Salze. Bevorzugt ist im allgemeinen jeweils die freie Form.

Die vor- und nachstehend verwendeten Allgemeinbegriffe haben, sofern nicht abweichend definiert, die nachfolgend aufgeführten Bedeutungen.

Halogen - als Gruppe per se sowie als Strukturelement von anderen Gruppen und Verbindungen, wie von Halogenalkyl und Halogenalkoxy, - ist Fluor, Chlor, Brom oder Iod, insbesondere Fluor, Chlor oder Brom, vor allem Fluor oder Chlor, insbesondere Chlor.

Kohlenstoffhaltige Gruppen und Verbindungen enthalten, sofern nicht abweichend definiert, jeweils 1 bis und mit 6, vorzugsweise 1 bis und mit 3, insbesondere 1 oder 2, Kohlenstoffatome.

Cycloalkyl ist Cyclopropyl, Cyclobutyl, Cyclopentyl oder Cyclohexyl, vorzugsweise Cyclopropyl.

Alkyl - als Gruppe per se sowie als Strukturelement von anderen Gruppen und Verbindungen, wie von Phenylalkyl, Halogenalkyl, Alkoxy und Halogenalkoxy, - ist, jeweils unter gebührender Berücksichtigung der von Fall zu Fall umfassten Anzahl der in der entsprechenden Gruppe oder Verbindung enthaltenen Kohlenstoffatome, entweder geradkettig, d. h. Methyl, Ethyl, Propyl, Butyl, Pentyl oder Hexyl, oder verzweigt, z. B. Isopropyl, Isobutyl, sek.-Butyl, tert.-Butyl, Isopentyl, Neopentyl oder Isohexyl.

Alkenyl und Alkinyl sind geradkettig oder verzweigt und enthalten jeweils zwei oder vorzugsweise eine ungesättigte Kohlenstoff-Kohlenstoff-Bindung(en). Die Doppel- oder Dreifachbindungen dieser Substituenten sind von dem restlichen Teil der Verbindung I vorzugsweise durch mindestens ein gesättigtes Kohlenstoffatom getrennt. Beispielhaft genannt seien Allyl, Methallyl, But-2-enyl, But-3-enyl, Propargyl, But 2-inyl und But-3-inyl.

Halogensubstituierte kohlenstoffhaltige Gruppen und Verbindungen, wie Halogenalkyl und Halogenalkoxy, können teilweise halogeniert oder perhalogeniert sein, wobei im Falle von Mehrfach-Halogenierung die Halogensubstituenten gleich oder verschieden sein können. Beispiele für Halogenalkyl - als Gruppe per se sowie als Strukturelement von anderen Gruppen und Verbindungen, wie von Halogenalkoxy, - sind das ein- bis dreifach durch Fluor, Chlor und/oder Brom substituierte Methyl, wie CHF₂ oder CF₃; das ein- bis fünffach durch Fluor, Chlor und/oder Brom substituierte Ethyl, wie CH₂CF₃, CF₂CF₃, CF₂CCl₃, CF₂CHCl₂, CF₂CHF₂, CF₂CFCl₂, CF₂CHBr₂, CF₂CHClF, CF₂CHBrF oder CClFCHClF; und das ein- bis siebenfach durch Fluor, Chlor und/oder Brom substituierte Propyl oder Isopropyl, wie CH₂CHBrCH₂Br, CF₂CHFCF₃, CH₂CF₂CF₃, CF₂CF₂CF₃ oder CH(CF₃)₂.

In Phenylalkyl ist eine an den restlichen Teil der Verbindung I gebundene Alkylgruppe durch eine Phenylgruppe substituiert, wobei die Alkylgruppe vorzugsweise geradkettig ist und die Phenylgruppe vorzugsweise in höherer als der α-Stellung, insbesondere in ω-Stellung, der Alkylgruppe gebunden ist; Beispiele sind Benzyl, 2-Phenylethyl und 4-Phenylbutyl.

Bevorzugte Ausführungsformen im Rahmen der Erfindung sind:
(1) Eine Verbindung der Formel I, worin
   A eine Pyridyl-, 1-Oxidopyridinio- oder Thiazolyl-Gruppe, die unsubstituiert oder ein- oder zweifach durch Substituenten, ausgewählt aus der Gruppe, bestehend aus Halogen, C₁-C₃-Alkyl, Halogen-C₁-C₃-alkyl, C₁-C₃-Alkoxy und Halogen-C₁-C₃-alkoxy, substituiert ist;
   R Wasserstoff, C₁-C₆-Alkyl, C₃-C₆-Cycloalkyl, C₂-C₆-Alkenyl oder C₂-C₆-Alkinyl; und X N-NO₂ oder N-CN bedeuten;
(2) Eine Verbindung der Formel I, worin das Ring-Grundgerüst von A eine Pyrid-3-yl-, 1-Oxido-3-pyridinio- oder Thiazol-5-yl-Gruppe, insbesondere A eine Pyrid-3-yl-, 2-Halogenpyrid-5-yl-, 2,3-Dihalogenpyrid-5-yl-, 2-C₁-C₃-Alkylpyrid-5-yl-, 1-Oxido-3-pyridinio-, 2-Halogen-1-oxido-5-pyridinio-, 2,3-Dihalogen-1-oxido-5-pyridinio- oder 2-Halogenthiazol-5-yl-Gruppe, vor allem A eine Pyrid-3-yl-, 2-Halogenpyrid-5-yl-, 2-Halogen-1-oxido-5-pyridinio- oder 2-Halogenthiazol-5-yl-Gruppe, vorzugsweise A eine 2-Chlorpyrid-5-yl-, 2-Methylpyrid-5-yl-, 1-Oxido-3-pyridinio-, 2-Chlor-1-oxido-5-pyridinio-, 2,3-Dichlor-1-oxido-5-pyridinio- oder 2-Chlorthiazol-5-yl-Gruppe, vor allem A eine Pyrid-3-yl-, 2-Chlorpyrid-5-yl-, 2-Chlor-1-oxido-5-pyridinio- oder 2-Chlorthiazol-5-yl-Gruppe, insbesondere A eine 2-Chlorpyrid-5-yl- oder vorzugsweise 2-Chlorthiazol-5-yl-Gruppe ist;
(3) Eine Verbindung der Formel I, worin R C₁-C₆-Alkyl, Phenyl-C₁-C₄-alkyl, C₃-C₆-Cycloalkyl, C₃-C₄-Alkenyl oder C₃-C₄-Alkinyl, vor allem C₁-C₆-Alkyl, C₃-C₆-Cycloalkyl, C₃-C₄-Alkenyl oder C₃-C₄-Alkinyl, vorzugsweise C₁-C₆-Alkyl, Phenyl-C₁-C₄-alkyl, C₃-C₄-Alkenyl oder C₃-C₄-Alkinyl, insbesondere C₁-C₄-Alkyl, vorzugsweise Methyl, ist;
(4) Eine Verbindung der Formel I, worin X N-NO₂ ist;
(5) Eine Verbindung der Formel I, worin A eine unsubstituierte oder ein- oder zweifach durch Substituenten, ausgewählt aus der Gruppe, bestehend aus Halogen und C₁-C₃-Alkyl, substituierte, Pyridyl-, 1-Oxidopyridinio- oder Thiazolyl-Gruppe, R C₁-C₆-Alkyl, Phenyl-C₁-C₄-alkyl, C₃-C₆-Cycloalkyl, C₃-C₄-Alkenyl oder C₃-C₄-Alkinyl und X N-NO₂ oder N-CN bedeuten;
(6) Eine Verbindung der Formel I, worin A eine 2-Chlorpyrid-5-yl-, 2-Methylpyrid-5-yl-, 1-Oxido-3-pyridinio-, 2-Chlor-1-oxido-5-pyridinio-, 2,3-Dichlor-1-oxido-5-pyridiniooder 2-Chlorthiazol-5-yl-Gruppe R C₁-C₄-Alkyl und X N-NO₂ bedeuten;
(7) Eine Verbindung der Formel I, worin A eine 2-Chlorthiazol-5-yl- oder 2-Chlorpyrid-5-yl-Gruppe, R C₁-C₄-Alkyl und X N-NO₂ bedeuten.

Besonders bevorzugt sind im Rahmen der Erfindung die in den Beispielen H3 und H4 genannten Verbindungen der Formel I.

Namentlich bevorzugt sind im Rahmen der Erfindung 5-(2-Chlorpyrid-5-ylmethyl)-3-methyl-4-nitroimino-perhydro-1,3,5-oxadiazin, 5-(2-Chlorthiazol-5-ylmethyl)-3-methyl-4-nitroimino-perhydro-1,3,5-oxadiazin, 3-Methyl-4-nitroimino-5-(1-oxido-3-pyridiniomethyl)-perhydro-1,3,5-oxadiazin, 5-(2-Chlor-1-oxido-5-pyridiniomethyl)-3-methyl-4-nitroimino-perhydro-1,3,5-oxadiazin und
3-Methyl-5-(2-methylpyrid-5-ylmethyl)-4-nitroimino-perhydro-1,3,5-oxadiazin.

Als weiterer Gegenstand der Erfindung ist das Verfahren zur Herstellung der Verbindungen der Formel I oder gegebenenfalls ihrer Tautomeren, jeweils in freier Form oder in Salzform, z. B. dadurch gekennzeichnet, dass man
a) eine Verbindung der Formel 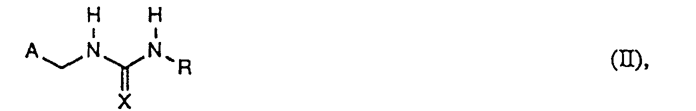 die bekannt ist oder in Analogie zu entsprechenden bekannten Verbindungen hergestellt werden kann und worin A, R und X die für die Formel I angegebenen Bedeutungen haben, oder ein Tautomeres und/oder Salz davon, vorzugsweise in Gegenwart einer Base oder ferner eines Säurekatalysators, mit Formaldehyd oder Paraformaldehyd umsetzt oder
b) zur Herstellung einer Verbindung der Formel I, worin R von Wasserstoff verschieden ist, oder eines Salzes davon eine, z. B. gemäss der Variante a) oder c) erhältliche, Verbindung der Formel I, worin R Wasserstoff ist, oder ein Tautomeres und/oder Salz davon, vorzugsweise in Gegenwart einer Base, mit einer Verbindung der Formel

   Y-R (III),

   die bekannt ist oder in Analogie zu entsprechenden bekannten Verbindungen hergestellt werden kann und worin R eine der für die Formel I angegebenen Bedeutungen mit Ausnahme von Wasserstoff hat und Y für eine Abgangsgruppe steht, umsetzt oder
c) eine Verbindung der Formel 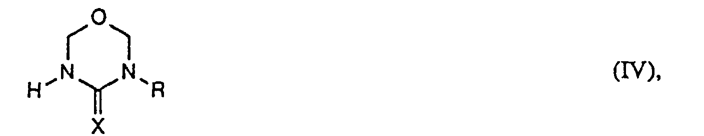
worin R und X die für die Formel I angegebenen Bedeutungen haben, oder ein Tautomeres und/oder Salz davon, vorzugsweise in Gegenwart einer Base, mit einer Verbindung der Formel

A-CH₂-Y (V),

die bekannt ist oder in Analogie zu entsprechenden bekannten Verbindungen hergestellt werden kann und worin A die für die Formel I angegebene Bedeutung hat und Y für eine Abgangsgruppe steht, oder einem Salz davon umsetzt und/oder, wenn erwünscht, eine verfahrensgemäss oder auf andere Weise erhältliche Verbindung der Formel I oder gegebenenfalls ein Tautomeres davon, jeweils in freier Form oder in Salzform, in eine andere Verbindung der Formel I oder gegebenenfalls ein Tautomeres davon überführt, ein verfahrensgemäss erhältliches Gemisch von isomeren auftrennt und das gewünschte Isomere isoliert und/oder eine verfahrensgemäss oder auf andere Weise erhältliche freie Verbindung der Formel I oder gegebenenfalls ein Tautomeres davon in ein Salz oder ein verfahrensgemäss oder auf andere Weise erhältliches Salz einer Verbindung der Formel I oder gegebenenfalls eines Tautomeren davon in die freie Verbindung der Formel I oder gegebenenfalls ein Tautomeres davon oder in ein anderes Salz überführt.

Für vor- und nachstehend aufgeführte Ausgangsmaterialien gilt im Hinblick auf deren Tautomere und/oder Salze das vorstehend für Tautomere und/oder Salze von Verbindungen I Gesagte in analoger Weise.

Die vor- und nachstehend beschriebenen Umsetzungen werden in an sich bekannter Weise durchgeführt, z. B. in Ab- oder üblicherweise in Anwesenheit eines geeigneten Lösungsoder Verdünnungsmittels oder eines Gemisches derselben, wobei man je nach Bedarf unter Kühlen, bei Raumtemperatur oder unter Erwärmen, z. B. in einem Temperaturbereich von etwa -80°C bis zur Siedetemperatur des Reaktionsgemisches, vorzugsweise von etwa -20°C bis etwa +150°C, und, falls erforderlich, in einem geschlossenen Gefäss, unter Druck, in einer Inertgasatmosphäre und/oder unter wasserfreien Bedingungen arbeitet. Besonders vorteilhafte Reaktionsbedingungen können den Beispielen entnommen werden.

Die vor- und nachstehend aufgeführten Ausgangsmaterialien, die für die Herstellung der Verbindungen I oder gegebenenfalls ihrer Tautomeren, jeweils in freier Form oder in Salzform, verwendet werden, sind bekannt oder können nach an sich bekannten Methoden, z. B. gemäss den nachstehenden Angaben, hergestellt werden.

### Variante a):

Geeignete Basen zur Erleichterung der Umsetzung sind z. B. Alkalimetall- oder Erdalkalimetall-hydroxide, -hydride, -amide, -alkanolate, -acetate, -carbonate, - dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natrium-hydroxid, - hydrid, -amid, -methanolat, -acetat, -carbonat, Kalium-tert.-butanolat, -hydroxid, - carbonat, -hydrid, Lithiumdiisopropylamid, Kalium-bis(trimethylsilyl)-amid, Calciumhydrid, Triethylamin, Diisopropyl-ethyl-amin, Triethylendiamin, Cyclohexylamin, N-Cyclohexyl-N,N-dimethyl-amin, N,N-Diethylanilin, Pyridin, 4-(N,N-Dimethylamino)pyridin, Chinuclidin, N-Methylmorpholin, Benzyl-trimethylammoniumhydroxid sowie 1,5-Diazabicyclo[5.4.0]undec-5-en (DBU) genannt.

Geeignete Säurekatalysatoren zur Erleichterung der Umsetzung sind z. B. diejenigen Säuren, eingesetzt in katalytischen Mengen, die vorstehend als für die Bildung von Säureadditionssalzen mit Verbindungen I geeignet aufgeführt sind.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Düsopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Alkohole, wie Methanol, Ethanol, Propanol, Isopropanol, Butanol, Ethylenglykol oder Glycerin; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid. Wird die Umsetzung in Gegenwart einer Base ausgeführt, können auch im Ueberschuss eingesetzte Basen, wie Triethylamin, Pyridin, N-Methylmorpholin oder N,N-Diethylanilin, als Lösungs- oder Verdünnungsmittel dienen. Wird die Umsetzung in Gegenwart eines Säurekatalysators ausgeführt, können auch im Ueberschuss eingesetzte Säuren, z. B. starke organische Carbonsäuren, wie gegebenenfalls, z. B. durch Halogen, substituierte C₁-C₄-Alkancarbonsäuren, z. B. Ameisensäure, Essigsäure oder Propionsäure, als Lösungs- oder Verdünnungsmittel dienen.

Die Umsetzung erfolgt vorteilhaft in einem Temperaturbereich von etwa 0°C bis etwa +180°C, bevorzugt von etwa +10°C bis etwa +130°C, in vielen Fällen im Bereich zwischen der Raumtemperatur und der Rückflusstemperatur des Reaktionsgemisches.

Das bei der Umsetzung entstehende Reaktionswasser kann gegebenenfalls mittels eines Wasserabscheiders, durch azeotrope Destillation oder durch Zusatz von geeignetem Molekularsieb entfernt werden.

### Variante b):

Geeignete Abgangsgruppen Y in den Verbindungen III sind z. B. Hydroxy, C₁-C₈-Alkoxy, Halogen-C₁-C₈-alkoxy, C₁-C₈-Alkanoyloxy, Mercapto, C₁-C₈-Alkylthio, Halogen-C₁-C₈alkylthio, C₁-C₈-Alkansulfonyloxy, Halogen-C₁-C₈-alkansulfonyloxy, Benzolsulfonyloxy, Toluolsulfonyloxy und Halogen.

Geeignete Basen zur Erleichterung der HY-Abspaltung sind z. B. von der unter der Variante a) angegebenen Art.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Alkohole, wie Methanol, Ethanol, Propanol, Isopropanol, Butanol, Ethylenglykol oder Glycerin; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid. Wird die Umsetzung in Gegenwart einer Base ausgeführt, können auch im Ueberschuss eingesetzte Basen, wie Triethylamin, Pyridin, N-Methylmorpholin oder N,N-Diethylanilin, als Lösungs- oder Verdünnungsmittel dienen.

Die Umsetzung erfolgt vorteilhaft in einem Temperaturbereich von etwa 0°C bis etwa +180°C, bevorzugt von etwa +10°C bis etwa +130°C, in vielen Fällen im Bereich zwischen der Raumtemperatur und der Rückflusstemperatur des Reaktionsgemisches.

### Variante c):

Geeignete Abgangsgruppen Y in den Verbindungen V sind z. B. von der unter der Variante b) angegebenen Art.

Geeignete Basen zur Erleichterung der HY-Abspaltung sind z. B. von der unter der Variante a) angegebenen Art.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Geeignete Lösungs- oder Verdünnungsmittel sind z. B. von der unter der Variante b) angegebenen Art.

Die Umsetzung erfolgt vorteilhaft in einem Temperaturbereich von etwa -20°C bis etwa +180°C, bevorzugt von etwa +10°C bis etwa +100°C, in vielen Fällen im Bereich zwischen der Raumtemperatur und der Rückflusstemperatur des Reaktionsgemisches.

Die in der Verfahrensvariante c) als Edukte eingesetzten Verbindungen IV und deren Tautomere, jeweils in freier Form oder in Salzform, sind, mit Ausnahme der in Anspruch 22 durch die beiden dort angegebenen Massgaben aus dem Anspruchsumfang ausgeschlossenen 3 Verbindungen in freier Form sowie ihrer Tautomeren, neu und bilden ebenfalls einen Gegenstand der Erfindung. Besonders bevorzugt sind im Rahmen der Erfindung die in den Beispielen H1 und H2 genannten neuen Verbindungen der Formel IV und deren Tautomere.

Als weiterer Gegenstand der Erfindung ist das Verfahren zur Herstellung der neuen Verbindungen der Formel IV oder ihrer Tautomeren, jeweils in freier Form oder in Salzform, z. B. dadurch gekennzeichnet, dass man
d) eine Verbindung der Formel 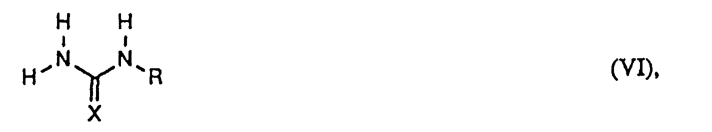 die bekannt ist oder in Analogie zu entsprechenden bekannten Verbindungen hergestellt werden kann und worin R und X die für die Formel IV angegebenen Bedeutungen haben, oder ein Tautomeres und/oder Salz davon mit Formaldehyd oder Paraformaldehyd, z. B. in analoger Weise wie unter der Variante a) für die entsprechende Umsetzung einer Verbindung der Formel II oder eines Tautomeren und/oder Salzes davon mit Formaldehyd oder Paraformaldehyd beschrieben, umsetzt oder
e) zur Herstellung einer Verbindung der Formel IV, worin R von Wasserstoff verschieden ist, oder eines Tautomeren und/oder Salzes davon eine, z. B. gemäss der Variante d) erhältliche, Verbindung der Formel IV, worin R Wasserstoff ist, oder ein Tautomeres und/oder Salz davon mit einer Verbindung der Formel

   Y-R (III),

   die bekannt ist oder in Analogie zu entsprechenden bekannten Verbindungen hergestellt werden kann und worin R eine der für die Formel IV angegebenen Bedeutungen mit Ausnahme von Wasserstoff hat und Y für eine Abgangsgruppe steht, z. B. in analoger Weise wie unter der Variante b) für die entsprechende Umsetzung einer Verbindung der Formel I, worin R Wasserstoff ist, oder eines Tautomeren und/oder Salzes davon mit einer Verbindung der Formel III beschrieben, umsetzt
   und/oder, wenn erwünscht, eine verfahrensgemäss oder auf andere Weise erhältliche Verbindung der Formel IV oder ein Tautomeres davon, jeweils in freier Form oder in Salzform, in eine andere Verbindung der Formel IV oder ein Tautomeres davon überführt, ein verfahrensgemäss erhältliches Gemisch von Isomeren auftrennt und das gewünschte Isomere isoliert und/oder eine verfahrensgemäss oder auf andere Weise erhältliche freie Verbindung der Formel IV oder ein Tautomeres davon in ein Salz oder ein verfahrensgemäss oder auf andere Weise erhältliches Salz einer Verbindung der Formel IV oder eines Tautomeren davon in die freie Verbindung der Formel IV oder ein Tautomeres davon oder in ein anderes Salz überführt.

Eine verfahrensgemäss oder auf andere Weise erhältliche Verbindung I bzw. IV kann in an sich bekannter Weise in eine andere Verbindung I bzw. IV überführt werden, indem man einen oder mehrere Substituenten der Ausgangsverbindung I bzw. IV in üblicher Weise durch (einen) andere(n) erfindungsgemässe(n) Substituenten ersetzt.

Beispielsweise können:
- in Verbindungen I mit unsubstituiertem Rest A Substituenten in den Rest A eingeführt werden; oder
- in Verbindungen I mit substituiertem Rest A Substituenten des Restes A durch andere Substituenten ersetzt werden.

Es ist dabei, je nach Wahl der dafür jeweils geeigneten Reaktionsbedingungen und Ausgangsmaterialien, möglich, in einem Reaktionsschritt nur einen Substituenten durch einen anderen erfindungsgemässen Substituenten zu ersetzen, oder es können in demselben Reaktionschritt zwei Substituenten durch andere erfindungsgemässe Substituenten ersetzt werden.

Salze von Verbindungen I bzw. IV können in an sich bekannter Weise hergestellt werden. So erhält man beispielsweise Säureadditionssalze von Verbindungen I bzw. IV durch Behandeln mit einer geeigneten Säure oder einem geeigneten Ionenaustauscherreagens und Salze mit Basen durch Behandeln mit einer geeigneten Base oder einem geeigneten Ionenaustauscherreagens.

Salze von Verbindungen I bzw. IV können in üblicher Weise in die freien Verbindungen I bzw. IV überführt werden, Säureadditionssalze z. B. durch Behandeln mit einem geeigneten basischen Mittel oder einem geeigneten Ionenaustauscherreagens und Salze mit Basen z. B. durch Behandeln mit einer geeigneten Säure oder einem geeigneten Ionenaustauscherreagens.

Salze von Verbindungen I bzw. IV können in an sich bekannter Weise in andere Salze von Verbindungen I bzw. IV umgewandelt werden, Säureadditionssalze beispielsweise in andere Säureadditionssalze, z. B. durch Behandeln eines Salzes einer anorganischen Säure, wie eines Hydrochlorids, mit einem geeigneten Metallsalz, wie einem Natrium-, Barium- oder Silbersalz, einer Säure, z. B. mit Silberacetat, in einem geeigneten Lösungsmittel, in welchem ein sich bildendes anorganisches Salz, z. B. Silberchlorid, unlöslich ist und damit aus dem Reaktionsgemisch ausscheidet.

Je nach Verfahrensweise bzw. Reaktionsbedingungen können die Verbindungen I bzw. IV mit salzbildenden Eigenschaften in freier Form oder in Form von Salzen erhalten werden.

Die Verbindungen I bzw. IV und jeweils gegebenenfalls ihre Tautomeren, jeweils in freier Form oder in Salzform, können in Form eines der möglichen Isomeren oder als Gemisch derselben, z. B. je nach Anzahl, absoluter und relativer Konfiguration von im Molekül auftretenden asymmetrischen Kohlenstoffatomen und/oder je nach Konfiguration von im Molekül auftretenden nichtaromatischen Doppelbindungen, als reine Isomere, wie Antipoden und/oder Diastereomere, oder als Isomerengemische, wie Enantiomerengemische, z. B. Racemate, Diastereomerengemische oder Racematgemische, vorliegen; die Erfindung betrifft sowohl die reinen Isomeren als auch alle möglichen Isomerengemische und ist vor- und nachstehend jeweils entsprechend zu verstehen, auch wenn stereochemische Einzelheiten nicht in jedem Fall speziell erwähnt werden.

Verfahrensgemäss - je nach Wahl der Ausgangsstoffe und Arbeitsweisen - oder anderweitig erhältliche Diastereomerengemische und Racematgemische von Verbindungen I bzw. IV, in freier Form oder in Salzform, können auf Grund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Entsprechend erhältliche Enantiomerengemische, wie Racemate, lassen sich nach bekannten Methoden in die optischen Antipoden zerlegen, beispielsweise durch Umkristallisation aus einem optisch aktiven Lösungsmittel, durch Chromatographie an chiralen Adsorbentien, z. B. Hochdruckflüssigkeitschromatographie (HPLC) an Acetylcellulose, mit Hilfe von geeigneten Mikroorganismen, durch Spaltung mit spezifischen, immobilisierten Enzymen, über die Bildung von Einschlussverbindungen, z. B. unter Verwendung chiraler Kronenether, wobei nur ein Enantiomeres komplexiert wird, oder durch Überführung in diastereomere Salze, z. B. durch Umsetzung eines basischen Endstoffracemats mit einer optisch aktiven Säure, wie Carbonsäure, z. B. Campher-, Wein- oder Äpfelsäure, oder Sulfonsäure, z. B. Camphersulfonsäure, und Trennung des auf diese Weise erhaltenen Diastereomerengemisches, z. B. auf Grund ihrer verschiedenen Löslichkeiten durch fraktionierte Kristallisation, in die Diastereomeren, aus denen das gewünschte Enantiomere durch Einwirkung geeigneter, z. B. basischer, Mittel freigesetzt werden kann.

Ausser durch Auftrennung entsprechender Isomerengemische können reine Diastereomere bzw. Enantiomere erfindungsgemäss auch durch allgemein bekannte Methoden der diastereoselektiven bzw. enantioselektiven Synthese erhalten werden, z. B. indem man das erfindungsgemässe Verfahren mit Edukten mit entsprechend geeigneter Stereochemie ausführt.

Vorteilhaft isoliert bzw. synthetisiert man jeweils das biologisch wirksamere Isomere, z. B. Enantiomere oder Diastereomere, oder Isomerengemisch, z. B. Enantiomerengemisch oder Diastereomerengemisch, sofern die einzelnen Komponenten unterschiedliche biologische Wirksamkeit besitzen.

Die Verbindungen I bzw. IV, in freier Form oder in Salzform, können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Erfindung betrifft alle diejenigen Ausführungsformen des Verfahrens, nach denen man von einer auf irgendeiner Stufe des Verfahrens als Ausgangs- oder Zwischenprodukt erhältlichen Verbindung ausgeht und alle oder einige der fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I bzw. deren Salzen führen.

Die Erfindung betrifft insbesondere die in den Beispielen H1 bis H4 beschriebenen Herstellungsverfahren.

Erfindungsgemäss für die Herstellung der Verbindungen I bzw. ihrer Salze verwendete Ausgangsstoffe und Zwischenprodukte, jeweils in freier Form oder in Salzform, die neu sind, ein Verfahren zu ihrer Herstellung und ihre Verwendung als Ausgangsstoffe und Zwischenprodukte für die Herstellung der Verbindungen I sind ebenfalls Gegenstand der Erfindung; insbesondere betrifft dies die Verbindungen IV.

Die erfindungsgemässen Verbindungen I sind auf dem Gebiet der Schädlingsbekämpfung bei günstiger Warmblüter-, Fisch- und Pflanzenverträglichkeit bereits bei niedrigen Anwendungskonzentrationen präventiv und/oder kurativ wertvolle Wirkstoffe mit einem sehr günstigen bioziden Spektrum. Die erfindungsgemässen Wirkstoffe sind gegen alle oder einzelne Entwicklungsstadien von normal sensiblen, aber auch von resistenten, tierischen Schädlingen, wie Insekten, wirksam. Die insektizide Wirkung der erfindungsgemässen Wirkstoffe kann sich dabei direkt, d. h. in einer Abtötung der Schädlinge, welche unmittelbar oder erst nach einiger Zeit, beispielsweise bei einer Häutung, eintritt, oder indirekt, z. B. in einer verminderten Eiablage und/oder Schlupfrate, zeigen, wobei die gute Wirkung einer Abtötungsrate (Mortalität) von mindestens 50 bis 60% entspricht.

Zu den erwähnten tierischen Schädlingen gehören beispielsweise: aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und
Trogoderma spp.;
aus der Ordnung Orthoptera zum Beispiel
Blatta spp., Blattella spp., Gryllotalpa spp., Leucophaea maderae, Locusta spp., Periplaneta spp. und Schistocerca spp.;
aus der Ordnung Isoptera zum Beispiel
Reticulitermes spp.;
aus der Ordnung Psocoptera zum Beispiel
Liposcelis spp.;
aus der Ordnung Anoplura zum Beispiel
Haematopinus spp., Linognathus spp., Pediculus spp., Pemphigus spp. und Phylloxera spp.;
aus der Ordnung Mallophaga zum Beispiel
Damalinea spp. und Trichodectes spp.;
aus der Ordnung Thysanoptera zum Beispiel
Frankliniella spp., Hercinothrips spp., Taeniothrips spp., Thrips palmi, Thrips tabaci und
Scirtothrips aurantii;
aus der Ordnung Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung Siphonaptera zum Beispiel
Ceratophyllus spp. und Xenopsylla cheopis und aus der Ordnung Thysanura zum Beispiel
Lepisma saccharina.

Mit den erfindungsgemässen Wirkstoffen kann man insbesondere an Pflanzen, vor allem an Nutz- und Zierpflanzen in der Landwirtschaft, im Gartenbau und im Forst, oder an Teilen, wie Früchten, Blüten, Laubwerk, Stengeln, Knollen oder Wurzeln, solcher Pflanzen auftretende Schädlinge des erwähnten Typus bekämpfen, d. h. eindämmen oder vernichten, wobei zum Teil auch später zuwachsende Pflanzenteile noch gegen diese Schädlinge geschützt werden.

Als Zielkulturen kommen insbesondere Getreide, wie Weizen, Gerste, Roggen, Hafer, Reis, Mais oder Sorghum; Rüben, wie Zucker- oder Futterrüben; Obst, z. B. Kern-, Steinund Beerenobst, wie Aepfel, Birnen, Pflaumen, Pfirsiche, Mandeln, Kirschen oder Beeren, z. B. Erdbeeren, Himbeeren oder Brombeeren; Hülsenfrüchte, wie Bohnen, Linsen, Erbsen oder Soja; Oelfrüchte, wie Raps, Senf, Mohn, Oliven, Sonnenblumen, Kokos, Rizinus, Kakao oder Erdnüsse; Gurkengewächse, wie Kürbisse, Gurken oder Melonen; Fasergewächse, wie Baumwolle, Flachs, Hanf oder Jute; Citrusfrüchte, wie Orangen, Zitronen, Pampelmusen oder Mandarinen; Gemüse, wie Spinat, Kopfsalat, Spargel, Kohlarten, Möhren, Zwiebeln, Tomaten, Kartoffeln oder Paprika; Lorbeergewächse, wie Avocado, Cinnamonium oder Kampfer; sowie Tabak, Nüsse, Kaffee, Eierfrüchte, Zuckerrohr, Tee, Pfeffer, Weinreben, Hopfen, Bananengewächse, Naturkautschukgewächse und Zierpflanzen in Betracht.

Die erfindungsgemässen Wirkstoffe eignen sich besonders zur Bekämpfung von Aphis craccivora, Bemisia tabaci, Diabrotica balteata, Heliothis virescens, Myzus persicae, Nephotettix cincticeps und Nilaparvata lugens in Gemüse-, Mais-, Obst-, Reis- und Soja-Kulturen.

Weitere Anwendungsgebiete der erfindungsgemässen Wirkstoffe sind der Schutz von Vorräten und Lagern und von'Material sowie im Hygienesektor insbesondere der Schutz von Haus- und Nutztieren vor Schädlingen des erwähnten Typus.

Die Erfindung betrifft daher auch Schädlingsbekämpfungsmittel, wie, je nach angestrebten Zielen und gegebenen Verhältnissen zu wählende, emulgierbare Konzentrate, Suspensionskonzentrate, direkt versprüh- oder verdünnbare Lösungen, streichfähige Pasten, verdünnte Emulsionen, Spritzpulver, lösliche Pulver, dispergierbare Pulver, benetzbare Pulver, Stäubemittel, Granulate oder Verkapselungen in polymeren Stoffen, welche-mindestens - einen der erfindungsgemässen Wirkstoffe enthalten.

Der Wirkstoff wird in diesen Mitteln in reiner Form, ein fester Wirkstoff z. B. in einer speziellen Korngrösse, oder vorzugsweise zusammen mit - mindestens - einem der in der Formulierungstechnik üblichen Hilfsstoffe, wie Streckmitteln, z. B. Lösungsmitteln oder festen Trägerstoffen, oder wie oberflächenaktiven Verbindungen (Tensiden), eingesetzt.

Als Lösungsmittel können z. B. in Frage kommen: gegebenenfalls partiell hydrierte aromatische Kohlenwasserstoffe, bevorzugt die Fraktionen C₈ bis C₁₂ von Alkylbenzolen, wie Xylolgemische, alkylierte Naphthaline oder Tetrahydronaphthalin, aliphatische oder cycloaliphatische Kohlenwasserstoffe, wie Paraffine oder Cyclohexan, Alkohole, wie Ethanol, Propanol oder Butanol, Glykole sowie deren Ether und Ester, wie Propylenglykol, Dipropylenglykolether, Ethylenglykol oder Ethylenglykolmono-methyl- oder - ethyl-ether, Ketone, wie Cyclohexanon, Isophoron oder Diacetanolalkohol, stark polare Lösungsmittel, wie N-Methylpyrrolid-2-on, Dimethylsulfoxid oder N,N-Dimethylformamid, Wasser, gegebenenfalls epoxidierte Pflanzenöle, wie gegebenenfalls epoxidiertes Raps-, Rizinus-, Kokosnuss- oder Sojaöl, und Silikonöle.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, und als nicht sorptive Trägermaterialien Calcit oder Sand in Frage. Darüberhinaus kann eine Vielzahl von granulierten Materialien anorganischer oder organischer Natur, insbesondere Dolomit oder zerkleinerte Pflanzenrückstände, verwendet werden.

Als oberflächenaktive Verbindungen kommen, je nach Art des zu formulierenden Wirkstoffs, nichtionische, kationische und/oder anionische Tenside oder Tensidgemische mit guten Emulgier-, Dispergier- und Netzeigenschaften in Betracht. Die nachstehend aufgeführten Tenside sind dabei nur als Beispiele anzusehen; in der einschlägigen Literatur werden viele weitere in der Formulierungstechnik gebräuchliche und erfindungsgemäss geeignete Tenside beschrieben.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignet sind wasserlösliche, 20 bis 250 Ethylenglykolether- und 10 bis 100 Propylenglykolether-gruppen enthaltende, Polyethylenoxid-Addukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykol-Einheiten. Als Beispiele seien Nonylphenolpolyethoxyethanole, Ricinusölpolyglykolether, Polypropylen-Polyethylenoxid-Addukte, Tributylphenoxypolyethoxyethanol, Polyethylenglykol und Octylphenoxypolyethoxyethanol erwähnt. Ferner kommen Fettsäureester von Polyoxyethylensorbitan, wie das Polyoxyethylensorbitan-trioleat, in Betracht.

Bei den kationischen Tensiden handelt es sich vor allem um quarternäre Ammoniumsalze, welche als Substituenten mindestens einen Alkylrest mit 8 bis 22 C-Atomen und als weitere Substituenten niedrige, gegebenenfalls halogenierte, Alkyl-, Benzyl- oder niedrige Hydroxyalkylreste aufweisen. Die Salze liegen vorzugsweise als Halogenide, Methylsulfate oder Ethylsulfate vor. Beispiele sind das Stearyl-trimethyl-ammoniumchlorid und das Benzyl-di-(2-chlorethyl)-ethyl-ammoniumbromid.

Geeignete anionische Tenside können sowohl wasserlösliche Seifen als auch wasserlösliche synthetische oberflächenaktive Verbindungen sein. Als Seifen eignen sich die Alkali-, Erdalkali- und gegebenenfalls substituierten Ammoniumsalze von höheren Fettsäuren (C₁₀-C₂₂), wie die Natrium- oder Kalium-Salze der Oel- oder Stearinsäure, oder von natürlichen Fettsäuregemischen, die beispielsweise aus Kokosnuss- oder Tallöl gewonnen werden können; ferner sind auch die Fettsäuremethyl-taurin-salze zu erwähnen. Häufiger werden jedoch synthetische Tenside verwendet, insbesondere Fettsulfonate, Fettsulfate, sulfonierte Benzimidazolderivate oder Alkylarylsulfonate. Die Fettsulfonate und -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst; beispielhaft genannt seien das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit etwa 8 bis 22 C-Atomen. Alkylarylsulfonate sind zum Beispiel die Natrium-, Calcium- oder Triethanolammoniumsalze der Dodecylbenzolsulfonsäure, der Dibutylnaphthalinsulfonsäure oder eines Naphthalinsulfonsäure-Formaldehyd-Kondensationsproduktes. Ferner kommen auch entsprechende Phosphate, wie Salze des Phosphorsäureesters eines p-Nonylphenol-(4-14)-Ethylenoxid-Adduktes oder Phospholipide, in Frage.

Die Mittel enthalten in der Regel 0,1 bis 99%, insbesondere 0,1 bis 95%, Wirkstoff und 1 bis 99,9%, insbesondere 5 bis 99,9%, - mindestens - eines festen oder flüssigen Hilfsstoffes, wobei in der Regel 0 bis 25%, insbesondere 0,1 bis 20%, der Mittel Tenside sein können (% bedeutet jeweils Gewichtsprozent). Während als Handelsware eher konzentrierte Mittel bevorzugt werden, verwendet der Endverbraucher in der Regel verdünnte Mittel, die wesentlich geringere Wirkstoffkonzentrationen aufweisen. Bevorzugte Mittel setzen sich insbesondere folgendermassen zusammen (% = Gewichtsprozent):

| Emulgierbare Konzentrate | |
|---|---|
| Wirkstoff | 1 bis 90%, vorzugsweise 5 bis 20% |
| Tensid | 1 bis 30%, vorzugsweise 10 bis20 % |
| Lösungsmittel | 5 bis 98%, vorzugsweise 70 bis 85% |

| Stäubemittel | |
|---|---|
| Wirkstoff | 0,1 bis 10%, vorzugsweise 0,1 bis 1 % |
| fester Trägerstoff | 99,9 bis 90%, vorzugsweise 99,9 bis 99% |

| Suspensionskonzentrate | |
|---|---|
| Wirkstoff | 5 bis 75%, vorzugsweise 10 bis 50% |
| Wasser | 94 bis 24%, vorzugsweise 88 bis 30% |
| Tensid | 1 bis 40%, vorzugsweise 2 bis 30% |

| Benetzbare Pulver | |
|---|---|
| Wirkstoff | 0,5 bis 90%, vorzugsweise 1 bis 80% |
| Tensid | 0,5 bis 20%, vorzugsweise 1 bis 15% |
| fester Trägerstoff | 5 bis 99%, vorzugsweise 15 bis 98% |

| Granulate | |
|---|---|
| Wirkstoff | 0,5 bis 30%, vorzugsweise 3 bis 15% |
| fester Trägerstoff | 99,5 bis 70%, vorzugsweise 97 bis 85% |

Die Wirkung der erfindungsgemässen Mittel lässt sich durch Zusatz von anderen insektiziden Wirkstoffen wesentlich verbreitern und an gegebene Umstände anpassen. Als Wirkstoff-Zusätze kommen dabei z. B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate. Die erfindungsgemässen Mittel können auch weitere feste oder flüssige Hilfsstoffe, wie Stabilisatoren, z. B. gegebenenfalls epoxidierte Pflanzenöle (z. B. epoxidiertes Kokosnussöl, Rapsöl oder Sojaöl), Entschäumer, z. B. Silikonöl, Konservierungsmittel, Viskositätsregulatoren, Bindemittel und/oder Haftmittel, sowie Düngemittel oder andere Wirkstoffe zur Erzielung spezieller Effekte, z. B. Akarizide, Bakterizide, Fungizide, Nematozide, Molluskizide oder selektive Herbizide, enthalten.

Die erfindungsgemässen Mittel werden in bekannter Weise hergestellt, bei Abwesenheit von Hilfsstoffen z. B. durch Mahlen, Sieben und/oder Presssen eines festen Wirkstoffs oder Wirkstoffgemisches, z. B. auf eine bestimmte Korngrösse, und bei Anwesenheit von mindestens einem Hilfsstoff z. B. durch inniges Vermischen und/oder Vermahlen des Wirkstoffs oder Wirkstoffgemisches mit dem (den) Hilfsstoff(en). Diese Verfahren zur Herstellung der erfindungsgemässen Mittel und die Verwendung der Verbindungen I zur Herstellung dieser Mittel bilden ebenfalls einen Gegenstand der Erfindung.

Die Anwendungsverfahren für die Mittel, also die Verfahren zur Bekämpfung von Schädlingen des erwähnten Typus, wie, je nach angestrebten Zielen und gegebenen Verhältnissen zu wählendes, Versprühen, Vernebeln, Bestäuben, Bestreichen, Beizen, Streuen oder Giessen, und die Verwendung der Mittel zur Bekämpfung von Schädlingen des erwähnten Typus sind weitere Gegenstände der Erfindung. Typische Anwendungskonzentrationen liegen dabei zwischen 0,1 und 1000 ppm, bevorzugt zwischen 0,1 und 500 ppm, Wirkstoff. Die Aufwandmengen pro Hektar betragen im allgemeinen 1 bis 2000 g Wirkstoff pro Hektar, insbesondere 10 bis 1000 g/ha, vorzugsweise 20 bis 600 g/ha.

Ein bevorzugtes Anwendungsverfahren auf dem Gebiet des Pflanzenschutzes ist das Aufbringen auf das Blattwerk der Pflanzen (Blattapplikation), wobei sich Applikationsfrequenz und Aufwandmenge auf den Befallsdruck des jeweiligen Schädlings ausrichten lassen. Der Wirkstoff kann aber auch durch das Wurzelwerk in die Pflanzen gelangen (systemische Wirkung), indem man den Standort der Pflanzen mit einem flüssigen Mittel tränkt oder den Wirkstoff in fester Form in den Standort der Pflanzen, z. B. in den Boden, einbringt, z. B. in Form von Granulat (Bodenapplikation). Bei Wasserreiskulturen kann man solche Granulate dem überfluteten Reisfeld zudosieren.

Die erfindungsgemässen Mittel eignen sich auch für den Schutz von pflanzlichem Vermehrungsgut, z. B. Saatgut, wie Früchten, Knollen oder Körnern, oder Pflanzenstecklingen, vor tierischen Schädlingen. Das Vermehrungsgut kann dabei vor dem Ausbringen mit dem Mittel behandelt, Saatgut z. B. vor der Aussaat gebeizt, werden. Die erfindungsgemässen Wirkstoffe können auch auf Samenkörner aufgebracht werden (Coating), indem man die Körner entweder in einem flüssigen Mittel tränkt oder sie mit einem festen Mittel beschichtet. Das Mittel kann auch beim Ausbringen des Vermehrungsguts auf den Ort der Ausbringung, z. B. bei der Aussaat in die Saatfurche, appliziert werden. Diese Behandlungsverfahren für pflanzliches Vermehrungsgut und das so behandelte pflanzliche Vermehrungsgut sind weitere Gegenstände der Erfindung.

Die folgenden Beispiele dienen der Erläuterung der Erfindung. Sie schränken die Erfindung nicht ein. Temperaturen sind in Grad Celsius angegeben.

### Herstellungsbeispiele

Beispiel H1: 3-Methyl-4-nitroimino-perhydro-1,3,5-oxadiazin 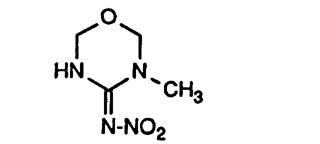 bzw. 3-Methyl-4-nitroamino-1,2,3,6-tetrahydro-1,3,5-oxadiazin

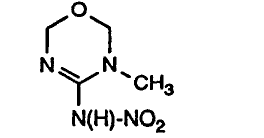

Ein Gemisch aus 20 g N-Methyl-N'-nitro-guanidin, 17 g Triethylamin, 100 ml Dioxan und 100 ml Toluol wird bei Raumtemperatur mit 30,5 g Paraformaldehyd versetzt, 16 Stunden unter Rückfluss erhitzt und dann im Vakuum eingedampft. Der Rückstand wird säulenchromatographisch [Kieselgel; Dichlormethan/Methanol (95:5)] gereinigt und liefert so die Titelverbindung, die bei 137 bis 139° schmilzt

Beispiel H2: In analoger Weise wie in Beispiel H1 beschrieben kann man auch herstellen:
3-Ethyl-4-nitroimino-perhydro-1,3,5-oxadiazin bzw. 3-Ethyl-4-nitroamino-1,2,3,6-tetrahydro-1,3,5-oxadiazin,
4-Nitroimino-3-propyl-perhydro-1,3,5-oxadiazin bzw. 4-Nitroamino-3-propyl-1,2,3,6-tetrahydro-1,3,5-oxadiazin (Harz),
3-Butyl-4-nitroimulo-perhydro-1,3,5-oxadiazin bzw. 3-Butyl-4-nitroamino-1,2,3,6-tetrahydro-1,3,5-oxadiazin (Schmelzpunkt: 80-82°),
3-Cyclopropyl-4-nitroimino-perhydro-1,3,5-oxadiazin bzw. 3-Cyclopropyl-4-nitroamino-1,2,3,6-tetrahydro-1,3,5-oxadiazin,
3-Allyl-4-nitroimino-perhydro-1,3,5-oxadiazin bzw. 3-Allyl-4-nitroamino-1,2,3,6-tetrahydro-1,3,5-oxadiazin (Harz),
4-Nitroimino-3-propargyl-perhydro-1,3,5-oxadiazin bzw. 4-Nitroamino-3-propargyl-1,2,3,6-tetrahydro-1,3,5-oxadiazin (Schmelzpunkt: 102-104°),
4-Cyanoimino-3-methyl-perhydro-1,3,5-oxadiazin bzw. 4-Cyanoamino-3-ethyl-1,2,3,6-tetrahydro-1,3,5-oxadiazin,
4-Cyanoimino-3-cyclopropyl-perhydro-1,3,5-oxadiazin bzw. 4-Cyanoamino-3-cyclopropyl-1,2,3,6-tetahydro-1,3,5-oxadiazin und
4-Nitroimino-3-(2-phenylethyl)-perhydro-1,3,5-oxadiazin bzw. 4-Nitroamino-3-(2-phenylethyl)-1,2,3,6-tetrahydro-1,3,5-oxadiazin (Schmelzpunkt: 123-125°).

Beispiel H3: 5-(2-Chlorpyrid-5-ylmethyl)-3-methyl-4-nitroimino-perhydro-1,3,5-oxadiazin (Tabelle 1, Verbindung Nr. 1.2).

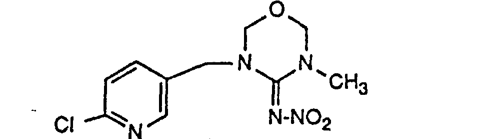

Ein Gemisch aus 1,44 g 3-Methyl-4-nitroimino-perhydro-1,3,5-oxadiazin, 2,2 g 2-Chlor-5-chlormethyl-pyridin, 3,7 g Kaliumcarbonat und 20 ml N,N-Dimethylformamid wird 4 Stunden auf 50° erhitzt, filtriert, das Filtrat am Rotationsverdampfer im Vakuum eingedampft und der Rückstand chromatographisch [Kieselgel; Dichlormethan/Methanol (95:5)] gereinigt. Man erhält so die Titelverbindung, die bei 116 bis 118° schmilzt.

Beispiel H4: In analoger Weise wie in den Beispielen H1 bis H3 beschrieben können auch die anderen in den Tabellen 1 und 2 aufgeführten Verbindungen hergestellt werden. In der Spalte "Physikalische Daten" dieser Tabellen bezeichnen die angegebenen Temperaturen jeweils den Schmelzpunkt der betreffenden Verbindung.

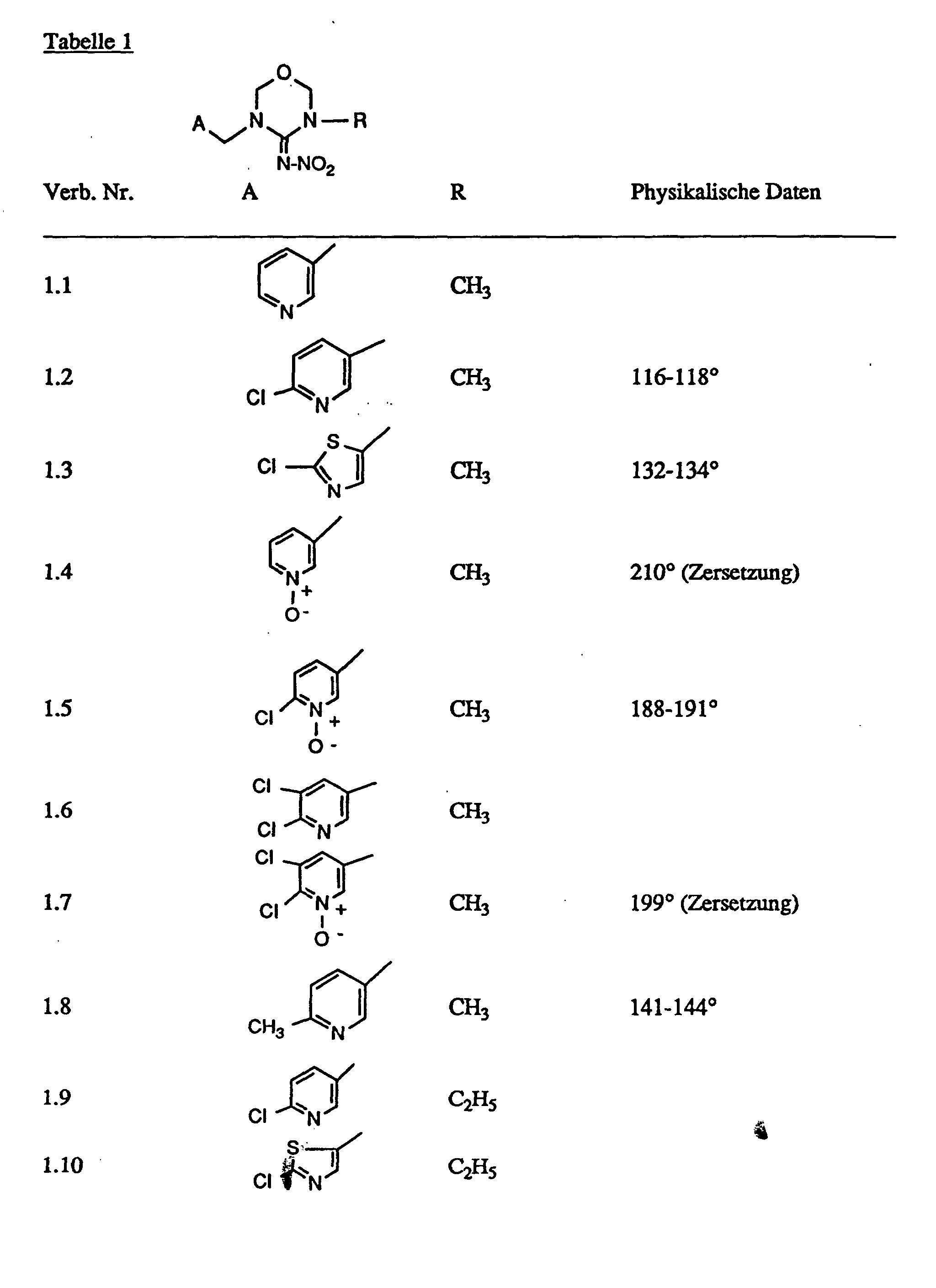

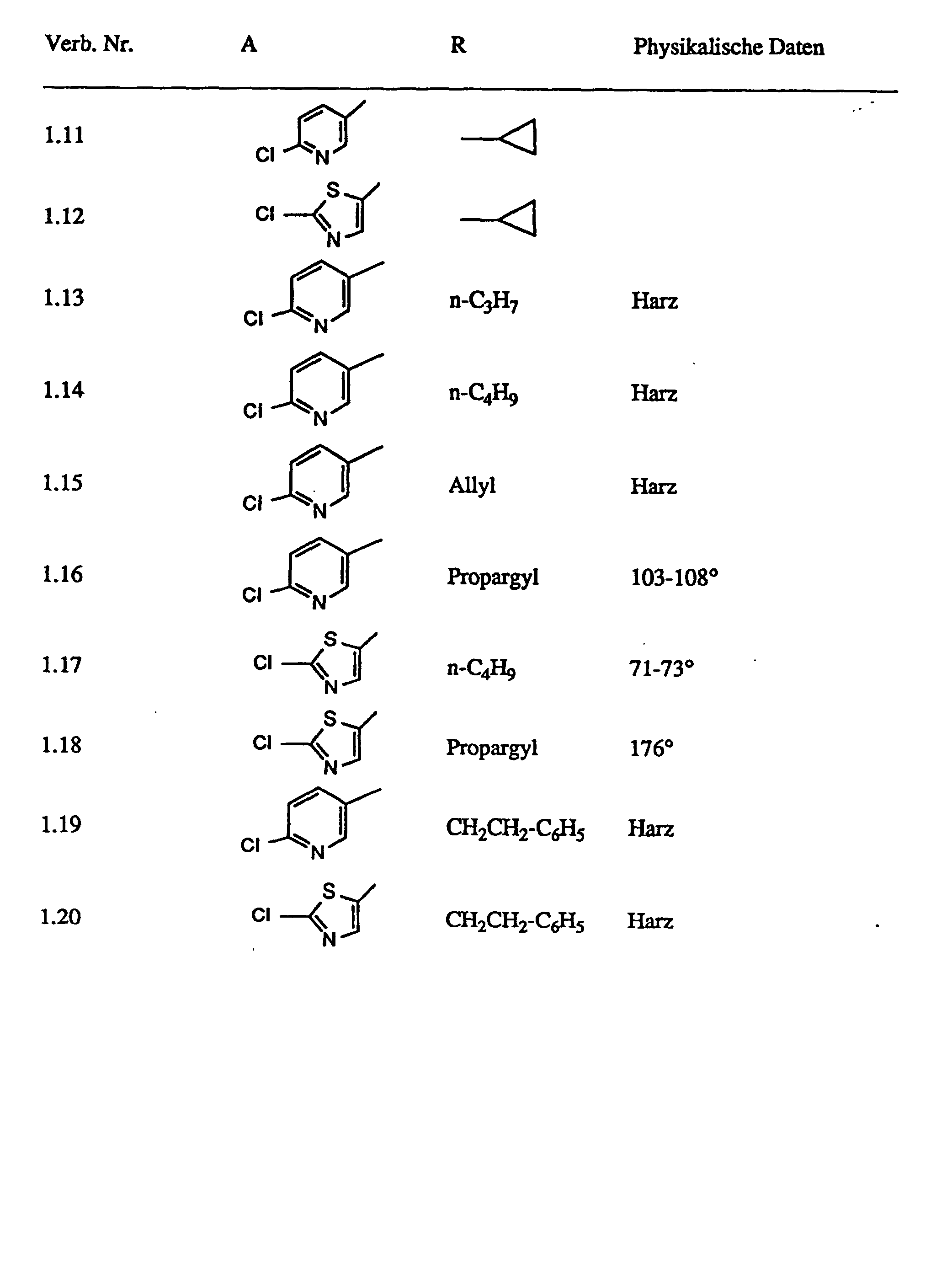

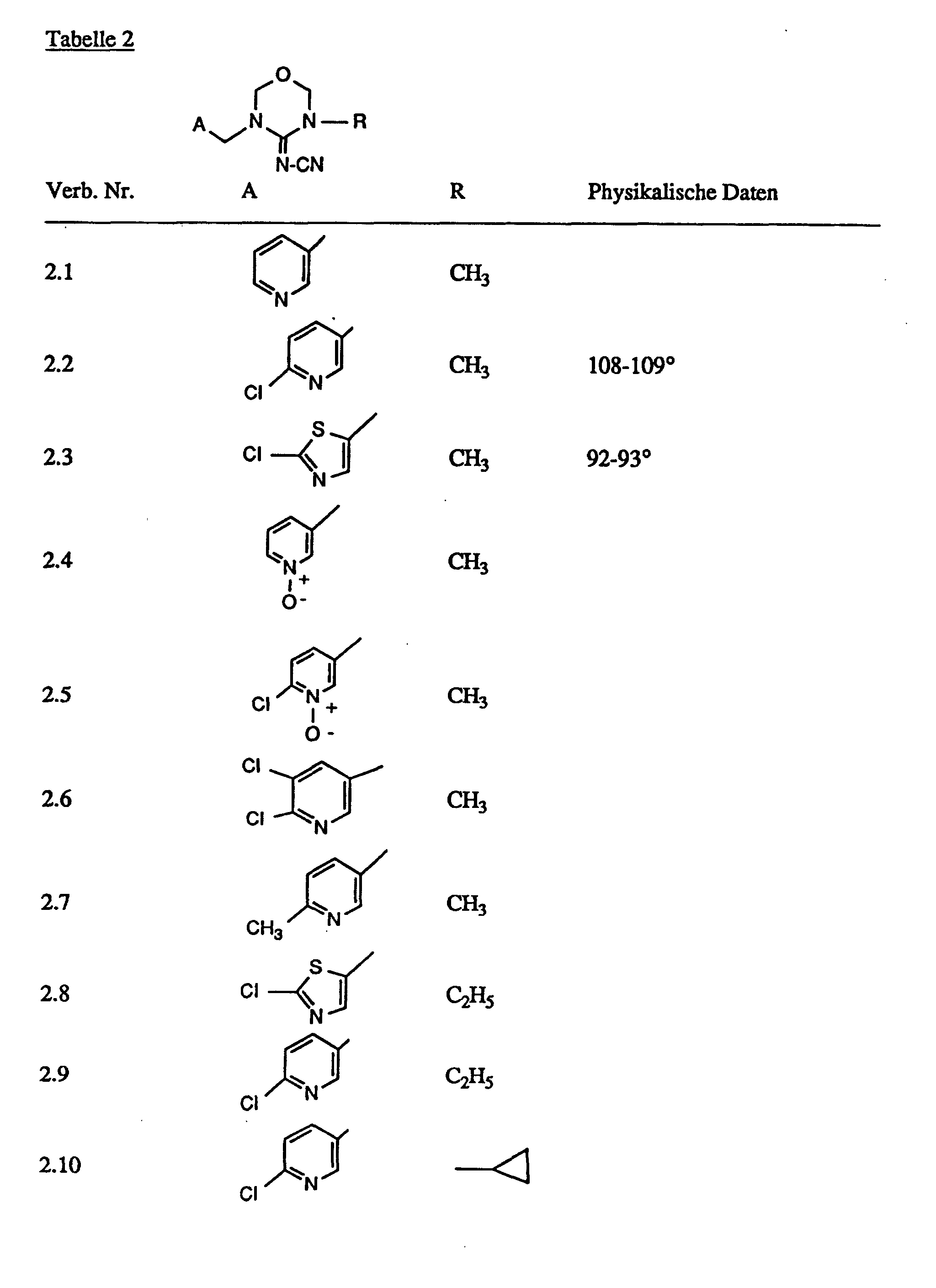

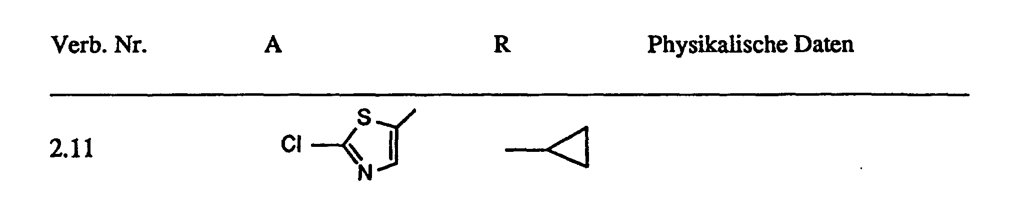

### Formulierungsbeispiele (% = Gewichtsprozent)

| Beispiel F1: Emulsions-Konzentrate | a) | b) | c) |
|---|---|---|---|
| Wirkstoff Nr. 1.2 | 25% | 40 % | 50 % |
| Ca-Dodecylbenzolsulfonat Ricinusölpolyethylenglykolether | 5 % | 8 % | 6 % |
| (36 Mol EO) Tributylphenolpolyethylenglykolether | 5 % | - | - |
| (30 Mol EO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff Nr. 1.3 | 80 % | 10 % | 5 % | 95 % |
| Ethylenglykolmonomethylether | 20 % | - | - | - |
| Polyethylenglykol MG 400 | - | 70 % | - | - |
| N-Methyl-2-pyrrolidon | - | 20 % | - | - |
| Epoxidiertes Kokosnussöl Benzin (Siedegrenzen | - | - | 1 % | 5 % |
| 160-190°C) | - | - | 94 % | - |

Die Lösungen sind zur Anwendung in Form kleinster Tropfen geeignet.

| Beispiel F3: Granulate | a) | b) | c) | d) |
|---|---|---|---|---|
| Wirkstoff Nr. 1.2 | 5 % | 10 % | 8% | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Dichlormethan gelöst, auf den Träger aufgesprüht und das Lösungsmittel anschliessend im Vakuum abgedampft.

| Beispiel F4: Stäubemittel | a) | b) |
|---|---|---|
| Wirkstoff Nr. 1.2 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff Nr. 1.2 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol EO) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

| Beispiel F6: Emulsions-Konzentrat | |
|---|---|
| Wirkstoff Nr. 1.3 Octylphenolpolyethylenglykolether | 10 % |
| (4-5 Mol EO) | 3 % |
| Ca-Dodecylbenzolsulfonat Ricinusölpolyglykolether | 3 % |
| (36 Mol EO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F7: Stäubemittel | a) | b) |
|---|---|---|
| Wirkstoff Nr. 1.2 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| Beispiel F8: Extruder-Granulat | |
|---|---|
| Wirkstoff Nr. 1.3 | 10 % |
| Na-Ligninsulfonat | 2 % |
| Carboxymethylcellulose | 1 % |
| Kaolin | 87% |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

| Beispiel F9: Umhüllungs-Granulat | |
|---|---|
| Wirkstoff Nr. 1.2 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| Beispiel F10: Suspensions-Konzentrat | |
|---|---|
| Wirkstoff Nr. 1.3 | 40 % |
| Ethylenglykol Nonylphenolpolyethylenglykolether | 10 % |
| (15 Mol EO) | 6 % |
| Na-Ligninsulfonat | 10 % |
| Carboxymethylcellulose | 1 % |
| 37%-ige wässrige Formaldehyd-Lösung Silikonöl in Form einer 75 %igen | 0,2 % |
| wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### Biologische Beispiele (% = Gewichtsprozent, sofern nichts anderes angegeben)

### Beispiel B1: Wirkung gegen Anthonomus grandis

Junge Baumwollpflanzen werden mit einer wässrigen Emulsionsspritzbrühe, die 400 ppm Wirkstoff enthält, besprüht. Nach dem Antrocknen des Spritzbelags werden die Pflanzen mit 10 Adulten von Anthonomus grandis besiedelt und in einen Plastikbehälter gegeben. 3 Tage später erfolgt die Auswertung. Aus den Vergleichen der Anzahl toter Käfer und des Frassschadens zwischen den behandelten und unbehandelten Pflanzen werden die prozentuale Reduktion der Population und die prozentuale Reduktion des Frassschadens (% Wirkung) bestimmt.
Verbindungen der Tabellen 1 und 2 zeigen gute Wirkung in diesem Test. Insbesondere zeigen die Verbindungen Nr. 1.2, 1.3 und 2.3 eine Wirkung von mehr als 80%.

### Beispiel B2: Wirkung gegen Aphis craccivora

Erbsenkeimlinge werden mit Aphis craccivora infiziert, anschliessend mit einer Spritzbrühe, die 400 ppm Wirkstoff enthält, besprüht und dann bei 20°C inkubiert. 3 und 6 Tage später erfolgt die Auswertung. Aus dem Vergleich der Anzahl toter Blattläuse auf den behandelten und auf unbehandelten Pflanzen wird die prozentuale Reduktion der Population (% Wirkung) bestimmt.
Verbindungen der Tabellen 1 und 2 zeigen gute Wirkung in diesem Test. Insbesondere zeigen die Verbindungen Nr. 1.2, 1.3, 1.15, 2.2 und 2.3 eine Wirkung von mehr als 80%.

### Beispiel B3: Wirkung gegen Bemisia tabaci

Buschbohnenpflanzen werden in Gazekäfige gestellt und mit Adulten von Bemisia tabaci besiedelt. Nach erfolgter Eiablage werden alle Adulten entfernt. 10 Tage später werden die Pflanzen mit den darauf befindlichen Nymphen mit einer wässrigen Emulsionsspritzbrühe, die 400 ppm Wirkstoff enthält, besprüht. Nach weiteren 14 Tagen wird der prozentuale Schlupf der Eier im Vergleich zu unbehandelten Kontrollansätzen ausgewertet.
Verbindungen der Tabellen 1 und 2 zeigen gute Wirkung in diesem Test. Insbesondere zeigen die Verbindungen Nr. 1.2 und 1.3 eine Wirkung von mehr als 80%.

### Beispiel B4: Wirkung gegen Ctenocephalides felis (systemisch)

Zwanzig adulte Flöhe der Art Ctenocephalides felis werden in einen flachen, runden, auf beiden Seiten mit Gaze verschlossenen, Käfig gegeben. Auf den Käfig wird ein Gefäss gestellt, das auf der Unterseite mit einer Parafilmmembran verschlossen ist. Das Gefäss enthält Blut, das 5 ppm Wirkstoff enthält und konstant auf 37° erwärmt wird. Die Flöhe nehmen das Blut durch die Membran auf. 24 und 48 Stunden nach Ansatz erfolgt die Auswertung. Aus dem Vergleich der Anzahl toter Flöhe bei Verwendung von behandeltem Blut und bei Verwendung von unbehandeltem Blut wird die prozentuale Reduktion der Population (% Wirkung) bestimmt. 24 Stunden nach Behandlung wird das Blut durch neues, ebenfalls behandeltes, Blut ersetzt.
Verbindungen der Tabellen 1 und 2 zeigen gute Wirkung in diesem Test. Insbesondere zeigen die Verbindungen Nr. 1.2 und 1.3 eine Wirkung von mehr als 80%.

### Beispiel B5: Wirkung gegen Diabrotica balteata

Maiskeimlinge werden mit einer wässrigen Emulsionsspritzbrühe, die 400 ppm Wirkstoff enthält, besprüht. Nach dem Antrocknen des Spritzbelags werden die Keimlinge mit 10 Larven des zweiten Stadiums von Diabrotica balteata besiedelt und in einen Plastikbehälter gegeben. 6 Tage später erfolgt die Auswertung. Aus dem Vergleich der Anzahl toter Larven zwischen den behandelten und unbehandelten Pflanzen wird die prozentuale Reduktion der Population (% Wirkung) bestimmt.
Verbindungen der Tabellen 1 und 2 zeigen gute Wirkung in diesem Test. Insbesondere zeigen die Verbindungen Nr. 1.2, 1.3, 1.5 und 2.3 eine Wirkung von mehr als 80%.

### Beispiel B6: Wirkung gegen Heliothis virescens

Junge Sojapflanzen werden mit einer wässrigen Emulsionsspritzbrühe, die 400 ppm Wirkstoff enthält, besprüht. Nach dem Antrocknen des Spritzbelages werden die Pflanzen mit 10 Raupen des ersten Stadiums von Heliothis virescens besiedelt und in einen Plastikbehälter gegeben. 6 Tage später erfolgt die Auswertung. Aus den Vergleichen der Anzahl toter Raupen und des Frassschadens zwischen den behandelten und unbehandelten Pflanzen werden die prozentuale Reduktion der Population und des Frasssschadens (% Wirkung) bestimmt.
Verbindungen der Tabellen 1 und 2 zeigen gute Wirkung in diesem Test. Insbesondere zeigen die Verbindungen Nr. 1.2 und 1.3 eine Wirkung von mehr als 80%.

### Beispiel B7: Wirkung gegen Heliothis virescens (ovi-/larvizid)

Auf Baumwolle abgelegte Eier von Heliothis virescens werden mit einer wässrigen Emulsionsspritzbrühe, die 400 ppm Wirkstoff enthält, besprüht. Nach 8 Tagen werden der prozentuale Schlupf der Eier und die Ueberlebensraten der Raupen im Vergleich zu unbehandelten Kontrollansätzen ausgewertet (% Reduktion der Population).
Verbindungen der Tabellen 1 und 2 zeigen gute Wirkung in diesem Test.

### Beispiel B8: Wirkung gegen Myzus persicae

Erbsenkeimlinge werden mit Myzus persicae infiziert, anschliessend mit einer Spritzbrühe, die 400 ppm Wirkstoff enthält, besprüht und dann bei 20° inkubiert. 3 und 6 Tage später erfolgt die Auswertung. Aus dem Vergleich der Anzahl toter Blattläuse auf den behandelten und auf unbehandelten Pflanzen wird die prozentuale Reduktion der Population (% Wirkung) bestimmt.
Verbindungen der Tabellen 1 und 2 zeigen gute Wirkung in diesem Test. Insbesondere zeigen die Verbindungen Nr. 1.2 und 1.3 eine Wirkung von mehr als 80%.

### Beispiel B9: Wirkung gegen Myzus persicae (systemisch)

Erbsenkeimlinge werden mit Myzus persicae infiziert, anschliessend mit den Wurzeln in eine Spritzbrühe, die 400 ppm Wirkstoff enthält, gestellt und dann bei 20° inkubiert. 3 und 6 Tage später erfolgt die Auswertung. Aus dem Vergleich der Anzahl toter Blattläuse auf den behandelten und auf unbehandelten Pflanzen wird die prozentuale Reduktion der Population (% Wirkung) bestimmt.
Verbindungen der Tabellen 1 und 2 zeigen gute Wirkung in diesem Test. Insbesondere zeigen die Verbindungen Nr. 1.2,1.3 und 1.5 eine Wirkung von mehr als 80%.

### Beispiel B10: Wirkung gegen Nephotettix cincticeps

Reispflanzen werden mit einer wässrigen Emulsionsspritzbrühe, die 400 ppm Wirkstoff enthält, besprüht. Nach dem Antrocknen des Spritzbelags werden die Pflanzen mit Larven des 2. und 3. Stadiums besiedelt. 21 Tage später erfolgt die Auswertung. Aus dem Vergleich der Anzahl überlebender Zikaden auf den behandelten und auf unbehandelten Pflanzen wird die prozentuale Reduktion der Population (% Wirkung) bestimmt. Verbindungen der Tabellen 1 und 2 zeigen gute Wirkung in diesem Test. Insbesondere zeigen die Verbindungen Nr. 1.2, 1.3 und 1.5 eine Wirkung von mehr als 80%.

### Beispiel B11: Wirkung gegen Nephotettix cincticeps (systemisch)

Töpfe mit Reispflanzen werden in eine wässrige Emulsionslösung, die 400 ppm Wirkstoff enthält, gestellt. Anschliessend werden die Pflanzen mit Larven des 2. und 3. Stadiums besiedelt. 6 Tage später erfolgt die Auswertung. Aus dem Vergleich der Anzahl Zikaden auf den behandelten und auf unbehandelten Pflanzen wird die prozentuale Reduktion der Population (% Wirkung) bestimmt.
Verbindungen der Tabellen 1 und 2 zeigen gute Wirkung in diesem Test. Insbesondere zeigen die Verbindungen Nr. 1.3, 1.5, 1.13 und 1.15 eine Wirkung von mehr als 80%.

### Beispiel B12: Wirkung gegen Nilaparvata lugens

Reispflanzen werden mit einer wässrigen Emulsionsspritzbrühe, die 400 ppm Wirkstoff enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten und auf unbehandelten Pflanzen wird die prozentuale Reduktion der Population (% Wirkung) bestimmt.
Verbindungen der Tabellen 1 und 2 zeigen gute Wirkung in diesem Test. Insbesondere zeigen die Verbindungen Nr. 1.2, 1.3, 1.5, 1.8 und 2.3 eine Wirkung von mehr als 80%.

### Beispiel B13: Wirkung gegen Nilaparvata lugens (systemisch)

Töpfe mit Reispflanzen werden in eine wässrige Emulsionslösung, die 10 ppm Wirkstoff enthält, gestellt. Anschliessend werden die Pflanzen mit Larven des 2. und 3. Stadiums besiedelt. 6 Tage später erfolgt die Auswertung. Aus dem Vergleich der Anzahl Zikaden auf den behandelten und auf unbehandelten Pflanzen wird die prozentuale Reduktion der Population (% Wirkung) bestimmt.
Verbindungen der Tabellen 1 und 2 zeigen gute Wirkung in diesem Test. Insbesondere zeigen die Verbindungen Nr. 1.2, 1.3, 1.4, 1.5, 1.13, 1.15, 2.2 und 2.3 eine Wirkung von mehr als 80%.

### Beispiel B14: Wirkung gegen Blattella germanica

In eine Petrischale wird so viel acetonische Lösung (0,1%) des Wirkstoffs gegeben, dass ihre Menge einer Aufwandmenge von 1 g/m² entspricht. Wenn das Lösungsmittel verdunstet ist, werden 10 Nymphen von Blattella germanica (letztes Nymphenstadium) in die Schale gegeben und 2 Stunden lang der Wirkung der Testsubstanz ausgesetzt. Dann werden die Nymphen mit CO₂ narkotisiert, in eine frische Petrischale gebracht und bei 25° und circa 70% Luftfeuchtigkeit im Dunkeln gehalten. Nach 48 Stunden wird die insektizide Wirkung durch Ermittlung der Abtötungsrate bestimmt.
Verbindungen der Tabellen 1 und 2 zeigen gute Wirkung in diesem Test. Insbesondere zeigt die Verbindung Nr. 1.3 eine Wirkung von mehr als 80%.

### Beispiel B15: Wirkung gegen Lucilia cuprina

Jeweils 30 bis 50 frisch abgelegte Eier von Lucilia cuprina werden in Reagensgläser gegeben, in denen zuvor 4 ml Nährmedium mit 1 ml Testlösung, die 16 ppm Wirkstoff enthält, vermischt worden sind. Nach Beimpfung des Kulturmediums werden die Testgläser mit einem Wattestopfen verschlossen und im Brutschrank 4 Tage bei 30° bebrütet. Im unbehandelten Medium entwickeln sich bis zu diesem Zeitpunkt ca. 1 cm lange Larven (Stadium 3). Ist die Testsubstanz aktiv, so sind die Larven zu diesem Zeitpunkt entweder tot oder deutlich zurückgeblieben. Die Auswertung erfolgt nach 96 Stunden.
Verbindungen der Tabellen 1 und 2 zeigen gute Wirkung in diesem Test. Insbesondere zeigt die Verbindung Nr. 1.3 eine Wirkung von mehr als 80%.

### Beispiel B 16: Wirkung gegen Musca domestica

Ein Zuckerwürfel wird mit so viel Testsubstanz-Lösung behandelt, dass nach Trocknen über Nacht die Konzentration der Testsubstanz im Zucker 250 ppm beträgt. Der so behandelte Würfel wird mit einem nassen Wattebausch und 10 Adulten eines OP-resistenten Stammes von Musca domestica auf eine Aluminiumschale gelegt. Diese wird mit einem Becherglas abgedeckt und bei 25° inkubiert. Nach 24 Stunden wird die Mortalitätsrate bestimmt.
Verbindungen der Tabellen 1 und 2 zeigen gute Wirkung in diesem Test. Insbesondere zeigt die Verbindung Nr. 1.3 eine Wirkung von mehr als 80%.

## Patentansprüche

1. Eine Verbindung der Formel 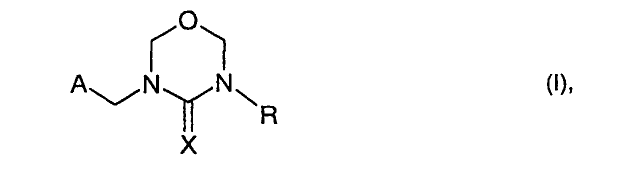 worin
A eine Pyridyl-, 1-Oxidopyridinio- oder Thiazolyl-Gruppe, die unsubstituiert oder ein- oder zweifach durch Substituenten, ausgewählt aus der Gruppe, bestehend aus Halogen, C₁-C₃-Alkyl, Halogen-C₁-C₃-alkyl, C₁-C₃-Alkoxy und Halogen-C₁-C₃-alkoxy, substituiert ist;
R Wasserstoff, C₁-C₆-Alkyl, Phenyl-C₁-C₄-alkyl, C₃-C₆-Cycloalkyl, C₂-C₆-Alkenyl oder C₂-C₆-Alkinyl; und
X N-NO₂ oder N-CN bedeuten, oder gegebenenfalls ein Tautomeres davon, jeweils in freier Form oder in Salzform.

2. Eine Verbindung gemäss Anspruch 1 der Formel I, worin R C₁-C₆-Alkyl, Phenyl-C₁-C₄alkyl, C₃-C₆-Cycloalkyl, C₃-C₄-Alkenyl oder C₃-C₄-Alkinyl ist.

3. Eine Verbindung gemäss Anspruch 1 der Formel I, worin X N-NO₂ ist, oder gegebenenfalls ein Tautomeres davon.

4. Eine Verbindung gemäss Anspruch 2 der Formel I, worin A eine unsubstituierte oder einoder zweifach durch Substituenten, ausgewählt aus der Gruppe, bestehend aus Halogen und C₁-C₃-Alkyl, substituierte, Pyridyl-, 1-Oxidopyridinio- oder Thiazolyl-Gruppe bedeutet.

5. Eine Verbindung gemäss Anspruch 4 der Formel I, worin A eine 2-Chlorpyrid-5-yl-, 2-Methylpyrid-5-yl-, 1-Oxido-3-pyridinio-, 2-Chlor-1-oxido-5-pyridinio-, 2,3-Dichlor-1-oxido-5-pyridinio- oder 2-Chlorthiazol-5-yl-Gruppe, R C₁-C₄-Alkyl und X N-NO₂ bedeuten.

6. Eine Verbindung gemäss Anspruch 4 der Formel I, worin A eine 2-Chlorthiazol-5-yl- oder 2-Chlorpyrid-5-yl-Gruppe und R C₁-C₄-Alkyl bedeuten.

7. Eine Verbindung gemäss Anspruch 6 der Formel I, worin X N-NO₂ bedeutet.

8. Eine Verbindung gemäss Anspruch 5 der Formel I, ausgewählt aus der Gruppe, bestehend aus den Verbindungen 5-(2-Chlorpyrid-5-ylmethyl)-3-methyl-4-nitroimino-perhydro-1,3,5-oxadiazin, 3-Methyl-4-nitroimino-5-(1-oxido-3-pyridiniomethyl)-perhydro-1,3,5-oxadiazin, 5-(2-Chlor-1-oxido-5-pyridiniomethyl)-3-methyl-4-nitroimino-perhydro-1,3,5-oxadiazin und 3-Methyl-5-(2-methylpyrid-5-ylmethyl)-4-nitroimino-perhydro-1,3,5-oxadiazin.

9. Eine Verbindung gemäss Anspruch 7 der Formel I, **dadurch gekennzeichnet, dass** es sich um 5-(2-Chlorthiazol-5-ylmethyl)-3-methyl-4-nitroimino-perhydro-1,3,5-oxadiazin handelt.

10. Verfahren zur Herstellung einer Verbindung gemäss Anspruch 1 der Formel I oder gegebenenfalls eines Tautomeren davon, jeweils in freier Form oder in Salzform, **dadurch gekennzeichnet, dass** man
a) eine Verbindung der Formel 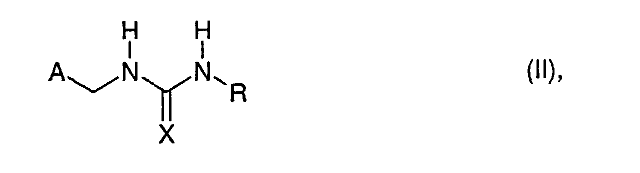 die bekannt ist oder in Analogie zu entsprechenden bekannten Verbindungen hergestellt werden kann und worin A, R und X die für die Formel I angegebenen Bedeutungen haben, oder ein Tautomeres und/oder Salz davon, vorzugsweise in Gegenwart einer Base oder ferner eines Säurekatalysators, mit Formaldehyd oder Paraformaldehyd umsetzt oder
b) zur Herstellung einer Verbindung der Formel I, worin R von Wasserstoff verschieden ist, oder eines Salzes davon eine, z. B. gemäss der Variante a) oder c) erhältliche, Verbindung der Formel I, worin R Wasserstoff ist, oder ein Tautomeres und/oder Salz davon, vorzugsweise in Gegenwart einer Base, mit einer Verbindung der Formel
Y―R (III),
die bekannt ist oder in Analogie zu entsprechenden bekannten Verbindungen hergestellt werden kann und worin R eine der für die Formel I angegebenen Bedeutungen mit Ausnahme von Wasserstoff hat und Y für eine Abgangsgruppe steht, umsetzt oder
c) eine Verbindung der Formel 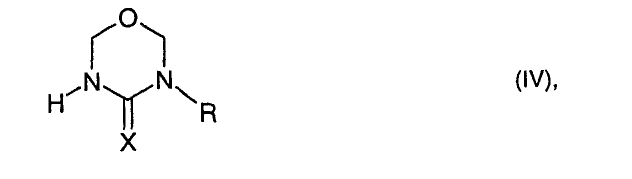 worin R und X die für die Formel I angegebenen Bedeutungen haben, oder ein Tautomeres und/oder Salz davon, vorzugsweise in Gegenwart einer Base, mit einer Verbindung der Formel
A―CH₂―Y (V),
die bekannt ist oder in Analogie zu entsprechenden bekannten Verbindungen hergestellt werden kann und worin A die für die Formel I angegebene Bedeutung hat und Y für eine Abgangsgruppe steht, oder einem Salz davon umsetzt
und/oder, wenn erwünscht, eine verfahrensgemäss oder auf andere Weise erhältliche Verbindung der Formel I oder gegebenenfalls ein Tautomeres davon, jeweils in freier Form oder in Salzform, in eine andere Verbindung der Formel I oder gegebenenfalls ein Tautomeres davon überführt, ein verfahrensgemäss erhältliches Gemisch von Isomeren auftrennt und das gewünschte Isomere isoliert und/oder eine verfahrensgemäss oder auf andere Weise erhältliche freie Verbindung der Formel I oder gegebenenfalls ein Tautomeres davon in ein Salz oder ein verfahrensgemäss oder auf andere Weise erhältliches Salz einer Verbindung der Formel I oder gegebenenfalls eines Tautomeren davon in die freie Verbindung der Formel I oder gegebenenfalls ein Tautomeres davon oder in ein anderes Salz überführt.

11. Schädlingsbekämpfungsmittel, **dadurch gekennzeichnet, dass** es mindestens eine Verbindung gemäss Anspruch 1 der Formel I oder gegebenenfalls ein Tautomeres davon, jeweils in freier Form oder in agrochemisch verwendbarer Salzform, als Wirkstoff und gegebenenfalls mindestens einen Hilfsstoff enthält.

12. Mittel gemäss Anspruch 11 zur Bekämpfung von Insekten.

13. Verfahren zur Herstellung eines mindestens einen Hilfsstoff enthaltenden Mittels gemäss Anspruch 11, **dadurch gekennzeichnet, dass** man den Wirkstoff mit dem (den) Hilfsstoff(en) innig vermischt und/oder vermahlt.

14. Verwendung einer Verbindung gemäss Anspruch 1 der Formel I oder gegebenenfalls eines Tautomeren davon, jeweils in freier Form oder in agrochemisch verwendbarer Salzform, zur Herstellung eines Mittels gemäss Anspruch 11.

15. Verwendung eines Mittels gemäss Anspruch 11 zur Bekämpfung von Schädlingen.

16. Verwendung gemäss Anspruch 15 zur Bekämpfung von Insekten.

17. Verwendung gemäss Anspruch 15 zum Schutz von pflanzlichem Vermehrungsgut.

18. Verfahren zur Bekämpfung von Schädlingen, **dadurch gekennzeichnet, dass** man ein Mittel gemäss Anspruch 11 auf die Schädlinge oder ihren Lebensraum appliziert.

19. Verfahren gemäss Anspruch 18 zur Bekämpfung von Insekten.

20. Verfahren gemäss Anspruch 18 zum Schutz von pflanzlichem Vermehrungsgut, **dadurch gekennzeichnet, dass** man das Vermehrungsgut oder den Ort der Ausbringung des Vermehrungsguts behandelt.

21. Pflanzliches Vermehrungsgut, behandelt gemäss dem in Anspruch 20 beschriebenen Verfahren.

22. Eine Verbindung der Formel 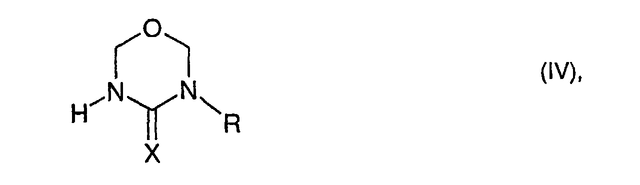 worin R und X die in Anspruch 1 für die Formel I angegebenen Bedeutungen haben, oder ein Tautomeres davon, jeweils in freier Form oder in Salzform, mit der Massgabe, dass in einer Verbindung der Formel IV oder einem Tautomeren davon, jeweils in freier Form, worin R Methyl bedeutet, X von N-CN verschieden ist, und mit der weiteren Massgabe, dass in einer Verbindung der Formel IV oder einem Tautomeren davon, jeweils in freier Form, worin X N-NO₂ oder N-CN ist, R von Wasserstoff verschieden ist.

23. Verfahren zur Herstellung einer Verbindung gemäss Anspruch 22 der Formel IV oder eines Tautomeren davon, jeweils in freier Form oder in Salzform, **dadurch gekennzeichnet, dass** man
d) eine Verbindung der Formel 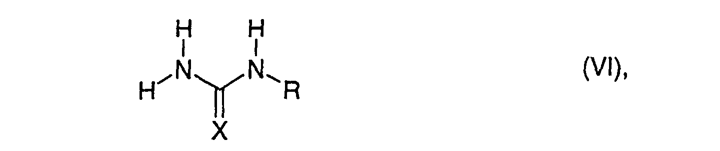 die bekannt ist oder in Analogie zu entsprechenden bekannten Verbindungen hergestellt werden kann und worin R und X die für die Formel IV angegebenen Bedeutungen haben, oder ein Tautomeres und/oder Salz davon mit Formaldehyd oder Paraformaldehyd, z. B. in analoger Weise wie unter der Variante a) für die entsprechende Umsetzung einer Verbindung der Formel II oder eines Tautomeren und/oder Salzes davon mit Formaldehyd oder Paraformaldehyd beschrieben, umsetzt oder
e) zur Herstellung einer Verbindung der Formel IV, worin R von Wasserstoff verschieden ist, oder eines Tautomeren und/oder Salzes davon eine, z. B. gemäss der Variante d) erhältliche, Verbindung der Formel IV, worin R Wasserstoff ist, oder ein Tautomeres und/oder Salz davon mit einer Verbindung der Formel
Y―R (III),
die bekannt ist oder in Analogie zu entsprechenden bekannten Verbindungen hergestellt werden kann und worin R eine der für die Formel IV angegebenen Bedeutungen mit Ausnahme von Wasserstoff hat und Y für eine Abgangsgruppe steht, z. B. in analoger Weise wie unter der Variante b) für die entsprechende Umsetzung einer Verbindung der Formel I, worin R Wasserstoff ist, oder eines Tautomeren und/oder Salzes davon mit einer Verbindung der Formel III beschrieben, umsetzt
und/oder, wenn erwünscht, eine verfahrensgemäss oder auf andere Weise erhältliche Verbindung der Formel IV oder ein Tautomeres davon, jeweils in freier Form oder in Salzform, in eine andere Verbindung der Formel IV oder ein Tautomeres davon überführt, ein verfahrensgemäss erhältliches Gemisch von Isomeren auftrennt und das gewünschte Isomere isoliert und/oder eine verfahrensgemäss oder auf andere Weise erhältliche freie Verbindung der Formel IV oder ein Tautomeres davon in ein Salz oder ein verfahrensgemäss oder auf andere Weise erhältliches Salz einer Verbindung der Formel IV oder eines Tautomeren davon in die freie Verbindung der Formel IV oder ein Tautomeres davon oder in ein anderes Salz überführt.

24. Verwendung einer Verbindung der Formel 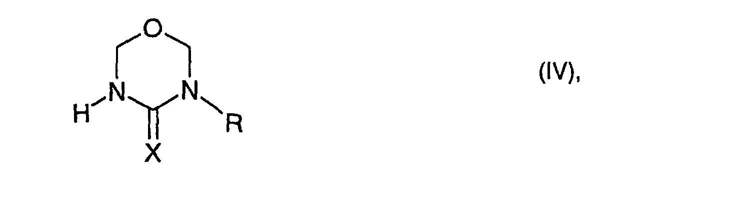 worin R und X die in Anspruch 1 für die Formel I angegebenen Bedeutungen haben, oder eines Tautomeren davon, jeweils in freier Form oder in Salzform, zur Herstellung einer Verbindung gemäss Anspruch 1 oder gegebenenfalls eines Tautomeren davon.

## Claims

1. A compound of formula 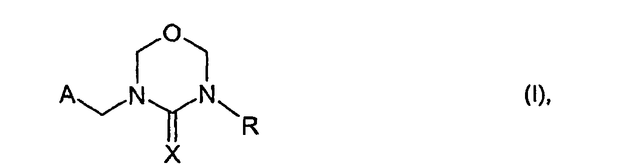 wherein
A is a pyridyl, 1-oxidopyridinio or thiazolyl group, which is unsubstituted or substituted once or twice by substituents selected from the group consisting of halogen, C₁-C₃alkyl, halo-C₁-C₃alkyl, C₁-C₃alkoxy and halo-C₁-C₃alkoxy;
R is hydrogen, C₁-C₆alkyl, phenyl-C₁-C₄alkyl, C₃-C₆cycloalkyl, C₂-C₆alkenyl or C₂-C₆alkynyl; and X is N-NO₂ or N-CN,
or, where applicable, a tautomer thereof, each in free form or in salt form.

2. A compound, according to claim 1, of formula I wherein R is C₁-C₆alkyl, phenyl-C₁-C₄alkyl, C₃-C₆cycloalkyl, C₃-C₄alkenyl or C₃-C₄alkynyl.

3. A compound, according to claim 1, of formula I wherein X is N-NO₂ or, where applicable, a tautomer thereof.

4. A compound, according to claim 2, of formula I wherein A is a pyridyl, 1-oxidopyridinio or thiazolyl group, which is unsubstituted or substituted once or twice by substituents selected from the group consisting of halogen and C₁-C₃alkyl.

5. A compound, according to claim 4, of formula I wherein A is a 2-chloropyrid-5-yl, 2-methylpyrid-5-yl, 1-oxido-3-pyridinio, 2-chloro-1-oxido-5-pyridinio, 2,3-dichloro-1-oxido-5-pyridinio or 2-chlorothiazol-5-yl group, R is C₁-C₄alkyl and X is N-NO₂.

6. A compound, according to claim 4, of formula I wherein A is a 2-chlorothiazol-5-yl or 2-chloropyrid-5-yl group and R is C₁-C₄alkyl.

7. A compound, according to claim 6, of formula I wherein X is N-NO₂.

8. A compound, according to claim 5, of formula I, selected from the group consisting of the compounds
5-(2-chloropyrid-5-ylmethyl)-3-methyl-4-nitroimino-perhydro-1,3,5-oxadiazine, 3-methyl-4-nitroimino-5-(1-oxido-3-pyridiniomethyl)-perhydro-1,3,5-oxadiazine, 5-(2-chloro-1-oxido-5-pyridiniomethyl)-3-methyl-4-nitroimino-perhydro-1,3,5-oxadiazine and 3-methyl-5-(2-methylpyrid-5-ylmethyl)-4-nitroimino-perhydro-1,3,5-oxadiazine.

9. A compound, according to claim 7, of formula I which is 5-(2-chlorothiazol-5-ylmethyl)-3-methyl-4-nitroimino-perhydro-1,3,5-oxadiazine.

10. A process for the preparation of a compound, according to claim 1, of formula I or, where applicable, a tautomer thereof, each in free form or in salt form, wherein
a) a compound of formula 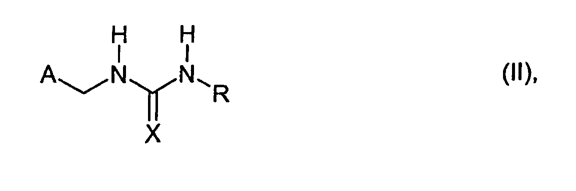 which is known or can be prepared in analogy to corresponding known compounds and in which A, R and X are as defined for formula I, or a tautomer and/or salt thereof, is reacted, preferably in the presence of a base or an acid catalyst, with formaldehyde or paraformaldehyde, or
b) for the preparation of a compound of formula I wherein R is other than hydrogen, or a salt thereof, a compound of formula I wherein R is hydrogen obtainable, for example, according to Variant a) or c), or a tautomer and/or salt thereof, is reacted, preferably in the presence of a base, with a compound of formula
Y―R (III),
which is known or can be prepared in analogy to corresponding known compounds and in which R is as defined for formula I with the exception of hydrogen and Y is a leaving group, or
c) a compound of formula 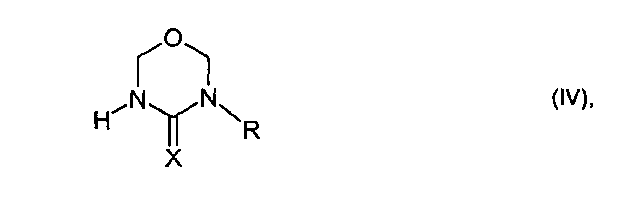 wherein R and X are as defined for formula I, or a tautomer and/or salt thereof, is reacted, preferably in the presence of a base, with a compound of formula
A―CH₂―Y (V),
which is known or can be prepared in analogy to corresponding known compounds and in which A is as defined for formula I and Y is a leaving group, or with a salt thereof, and/or, if desired, a compound of formula I obtainable according to the process or by some other method or, where applicable, a tautomer thereof, each in free form or in salt form, is converted into a different compound of formula I or, where applicable, into a tautomer thereof, a mixture of isomers obtainable according to the process is separated and the desired isomer is isolated, and/or a free compound of formula I obtainable according to the process or by some other method or, where applicable, a tautomer thereof is converted into a salt, or a salt, obtainable according to the process or by some other method, of a compound of formula I or, where applicable, of a tautomer thereof, is converted into the free compound of formula I or, where applicable, into a tautomer thereof, or into a different salt.

11. A pesticidal composition which comprises at least one compound, according to claim 1, of formula I or, where applicable, a tautomer thereof, each in free form or in agrochemically acceptable salt form, as active ingredient and, if desired, at least one adjuvant.

12. A composition according to claim 11 for controlling insects.

13. A process for the preparation of a composition according to claim 11 comprising at least one adjuvant, wherein the active ingredient is intimately mixed and/or ground with the adjuvant(s).

14. Use of a compound, according to claim 1, of formula I or, where applicable, a tautomer thereof, each in free form or in agrochemically acceptable salt form, in the preparation of a composition according to claim 11.

15. Use of a composition according to claim 11 in the control of pests.

16. Use according to claim 15 in the control of insects.

17. Use according to claim 15 in the protection of plant propagation material.

18. A method of controlling pests which comprises applying a composition according to claim 11 to the pests or to the locus thereof.

19. A method according to claim 18 for controlling insects.

20. A method according to claim 18 for protecting plant propagation material which comprises treating the propagation material or the area in which the propagation material is being planted.

21. Plant propagation material treated according to the method described in claim 20.

22. A compound of formula 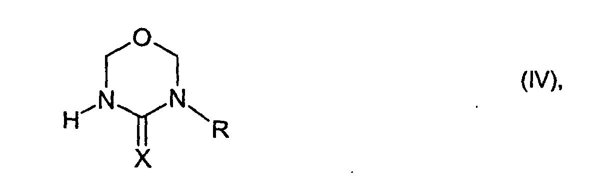 wherein R and X are as defined for formula in claim 1, or a tautomer thereof, each in free form or in salt form, with the proviso that in a compound of formula IV or a tautomer thereof, each in free form, in which R is methyl, X is other than N-CN, and with the further proviso that in a compound of formula IV or a tautomer thereof, each in free form, wherein X is N-NO₂ or N-CN, R is other than hydrogen.

23. A process for the preparation of a compound according to claim 22 of formula IV or a tautomer thereof, each in free form or in salt form, wherein
d) a compound of formula 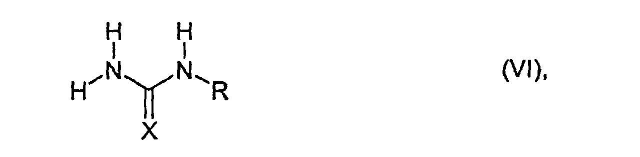 which is known or which can be prepared in analogy to corresponding known compounds, and in which R and X are as defined for formula IV, or a tautomer and/or salt thereof, is reacted with formaldehyde or paraformaldehyde, for example in a manner analogous to that described under Variant a) for the corresponding reaction of a compound of formula II or a tautomer and/or salt thereof with formaldehyde or paraformaldehyde, or
e) for the preparation of a compound of formula IV wherein R is other than hydrogen, or a tautomer and/or salt thereof, a compound of formula IV wherein R is hydrogen obtainable, for example, according to Variant d), or a tautomer and/or salt thereof, is reacted with a compound of formula
Y-R (III),
which is known or can be prepared in analogy to corresponding known compounds, and in which R is as defined for formula IV with the exception of hydrogen and Y is a leaving group, the reaction being carried out, for example, in a manner analogous to that described under Variant b) for the corresponding reaction of a compound of formula I wherein R is hydrogen or a tautomer and/or salt thereof with a compound of formula III,
and/or, if desired, a compound of formula IV obtainable according to the process or by some other method, or a tautomer thereof, each in free form or in salt form, is converted into a different compound of formula IV or into a tautomer thereof, a mixture of isomers obtainable according to the process is separated and the desired isomer is isolated, and/or a free compound of formula IV obtainable according to the process or by some other method, or a tautomer thereof, is converted into a salt, or a salt, obtainable according to the process or by some other method, of a compound of formula IV or of a tautomer thereof is converted into the free compound of formula IV or into a tautomer thereof or into a different salt.

24. The use of a compound of formula 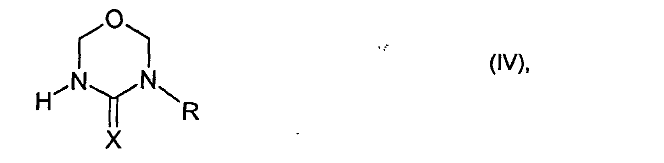 wherein R and X are as defined for formula I in claim 1, or a tautomer thereof, each in free form or in salt form, in the preparation of a compound according to claim 1 or, where applicable, a tautomer thereof.

## Revendications

1. Composé de formule : 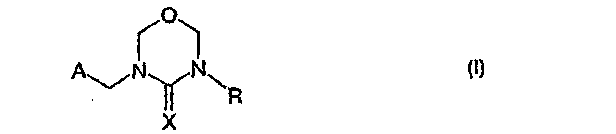 où
A représente un radical pyridyle, 1-oxydopyridino ou thiazolyle, qui est non substitué ou substitué une ou deux fois, par des substituants choisis dans le groupe consistant en halogène, alkyle en C₁-C₃, halogénoalkyle en C₁-C₃, alcoxy en C₁-C₃ et halogénoalcoxy en C₁-C₃ ; R représente hydrogène, alkyle en C₁-C₆, phénylalkyle en C₁-C₄, cycloalkyle en C₃-C₆, alcényle en C₂-C₆ ou alcynyle en C₂-C₆, et
X représente N-NO₂ ou N-CN, ou le cas échéant, un tautomère de celui-ci, chaque fois sous forme libre ou de sel.

2. Composé de formule I selon la revendication 1, où R représente alkyle en C₁-C₆, phénylalkyle en C₁-C₄, cycloalkyle en C₃-C₆, alcényle en C₃-C₄ ou alcynyle en C₃-C₄.

3. Composé de formule I selon la revendication 1, où X est N-NO₂, ou le cas échéant, un tautomère de celui-ci.

4. Composé de formule I selon la revendication 2, où A représente un radical pyridyle, 1-oxydopyridino ou thiazolyle, non substitué ou substitué une ou deux fois, par des substituants choisis dans le groupe consistant en halogène et alkyle en C₁-C₃.

5. Composé de formule I selon la revendication 4, où A représente le radical 2-chloropyrid-5-yle, 2-méthylpyrid-5-yle, 1-oxydo-3-pyridino, 2-chloro-1-oxydo-5-pyridino, 2,3-dichloro-1-oxydo-5-pyridino ou 2-chlorothiazol-5-yle, R représente alkyle en C₁-C₄, et X représente N-NO₂.

6. Composé de formule I selon la revendication 4, où A représente le radical 2-chlorothiazol-5-yle ou 2-chloropyrid-5-yle et R représente alkyle en C₁-C₄.

7. Composé de formule I selon la revendication 6, où X représente N-NO₂.

8. Composé de formule I selon la revendication 5, choisi parmi le groupe consistant en les composés :
la 5-(2-chloropyrid-5-ylméthyl)-3-méthyl-4-nitro-iminoperhydro-1,3,5-oxadiazine ;
la 3-méthyl-4-nitroimino-5-(1-oxydo-3-pyridino-méthyl)perhydro-1,3,5-oxadiazine ;
la 5-(2-chloro-1-oxydo-5-pyridinométhyl)-3-méthyl-4-nitro-iminoperhydro-1,3,5-oxadiazine, et
la 3-méthyl-5-(2-méthylpyrid-5-ylméthyl)-4-nitroiminoperhydro-1,3,5-oxadiazine.

9. Un composé de formule I selon la revendication 7, **caractérisé en ce qu'**il s'agit de la 5-(2-chlorothiazol-5-ylméthyl)-3-méthyl-4-nitroimino-perhydro-1,3,5-oxadiazine.

10. Procédé de préparation d'un composé de formule I selon la revendication 1 ou le cas échéant, d'un tautomère de celui-ci, chaque fois sous forme libre ou de sel, **caractérisé en ce que** :
a) on fait réagir un composé de formule : 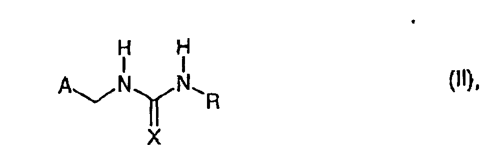 qui est connu ou peut etre prepare par analogie aux composés connus correspondants, et où A, R et X ont les significations indiquées à la formule I, ou d'un tautomère et/ou sel de celui-ci, de préférence en présence d'une base ou de plus, d'un catalyseur acide, avec du formaldéhyde ou du paraformaldéhyde, ou
b) pour la préparation d'un composé de formule I, où R est différent de hydrogène, ou d'un sel de celui-ci, on fait réagir un composé de formule I, qui peut être obtenu selon la variante a) ou c), où R est hydrogène, ou un tautomère et/ou sel de celui-ci, de préférence en présence d'une base, avec un composé de formule :
Y - R (III)
qui est connu ou peut être préparé par analogie aux composés connus correspondants, et où R a une des significations indiquées pour la formule I, à l'exception de hydrogène, et Y est un groupe partant, ou
c) on fait réagir un composé de formule : 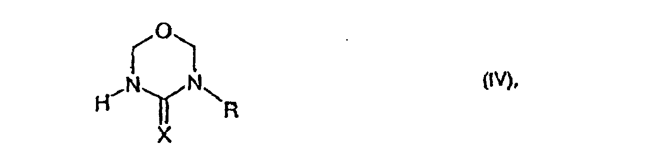 où R et X ont les significations indiquées pour la formule I, ou un tautomère et/ou sel de celui-ci, de préférence en présence d'une base, avec un composé de formule :
A -CH₂-Y (V)
qui est connu ou peut être préparé par analogie aux composés connus correspondants, et où A a la signification indiquée pour la formule I, et Y est un groupe partant, ou un sel de celui-ci,
et/ou si souhaité, un composé de formule I selon le procédé ou obtenu d'une autre manière, ou le cas échéant, un tautomère de celui-ci, chaque fois sous la forme libre ou d'un sel, est converti en un autre composé de formule I, ou le cas échéant, un tautomère de celui-ci, un mélange d'isomères obtenu selon le procédé est séparé et l'isomère souhaité est isolé et/ou un composé libre de formule I selon le procédé ou obtenu d'une autre manière, ou le cas échéant, un tautomère de celui-ci, est converti en un sel ou un sel obtenu selon le procédé ou d'une autre manière d'un composé de formule I ou le cas échéant, d'un tautomère de celui-ci, est transformé en le composé libre de formule I ou le cas échéant, d'un tautomère de celui-ci, ou en un autre sel.

11. Agent de lutte contre les parasites, **caractérisé en ce qu'**il contient au moins un composé de formule I selon la revendication 1, ou le cas échéant, un tautomère de celui-ci, chaque fois sous forme libre ou de sel utilisable en agronomie, comme agent actif et le cas échéant, au moins un auxiliaire.

12. Agent selon la revendication 11, pour lutter contre les insectes.

13. Procédé de préparation d'un agent contenant au moins un auxiliaire selon la revendication 11, **caractérisé en ce que** l'on mélange intimement et/ou broie l'agent actif avec le ou les auxiliaires.

14. Utilisation d'un composé de formule I selon la revendication 1, ou le cas échéant, un tautomère de celui-ci, chaque fois sous forme libre ou de sel utilisable en agronomie, pour la préparation d'un agent selon la revendication 11.

15. Utilisation d'un agent selon la revendication 11, pour lutter contre les parasites.

16. Utilisation selon la revendication 15, pour lutter contre les insectes.

17. Utilisation selon la revendication 15, pour la protection des produits végétaux de reproduction.

18. Procédé pour lutter contre les parasites, **caractérisé en ce que** l'on applique un agent selon la revendication 11, sur les parasites ou leur biotope.

19. Procédé selon la revendication 18, pour lutter contre les insectes.

20. Procédé selon la revendication 18, pour la protection des produits végétaux de reproduction, **caractérisé en ce que** l'on traite le produit de reproduction ou le site d'application du produit de reproduction.

21. Produit végétal de reproduction, traité selon le procédé décrit à la revendication 20.

22. Composé de formule : 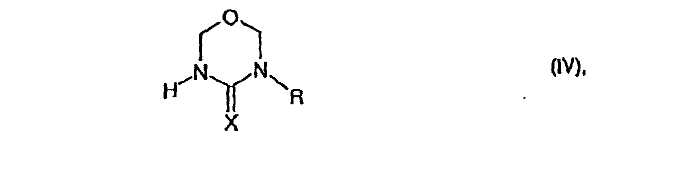 où R et X ont les significations indiquées pour la formule I à la revendication 1, ou un tautomère de celui-ci, chaque fois sous forme libre ou de sel, avec la condition que dans un composé de formule IV ou un tautomère de celui-ci, chaque fois sous forme libre, si R représente méthyle, X est différent de N-CN et avec la condition que dans un composé de formule IV ou un tautomère de celui-ci, chaque fois sous forme libre, si X est N-NO₂ ou N-CN, R est différent de hydrogène.

23. Procédé de préparation d'un composé de formule IV selon la revendication 22, ou d'un tautomère de celui-ci, chaque fois sous forme libre ou d'un sel, **caractérisé en ce que** :
d) on fait réagir un composé de formule : 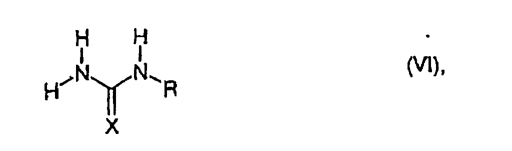 qui est connu ou peut etre prepare par analogie aux composés connus correspondants, et où R et X ont les significations indiquées pour la formule IV, ou d'un tautomère et/ou sel de celui-ci, avec du formaldéhyde ou du paraformaldéhyde, par exemple de manière analogue à ce qui a été décrit à la variante a) pour la réaction correspondante d'un composé de formule II ou d'un tautomère et/ou sel de celui-ci, avec du formaldéhyde ou du paraformaldéhyde, ou
e) pour la préparation d'un composé de formule IV,
où R est différent de hydrogène, ou d'un tautomère et/ou sel de celui-ci, on fait réagir un composé de formule IV, qui peut être obtenu selon la variante d), où R est hydrogène, ou un tautomère et/ou sel de celui-ci, avec un composé de formule :
Y - R (III)
qui est connu ou peut être préparé par analogie aux composés connus correspondants, et où R a une des significations indiquées pour la formule IV, à l'exception de hydrogène, et Y représente un groupe partant, par exemple de manière analogue à ce qui a été décrit à la variante b) pour la réaction correspondante d'un composé de formule I, où R est hydrogène, ou d'un tautomère et/ou sel de celui-ci avec un composé de formule III
et/ou si souhaité, un composé de formule IV selon le procédé ou obtenu d'une autre manière, ou un tautomère de celui-ci, chaque fois sous la forme libre ou d'un sel, est converti en un autre composé de formule IV, ou un tautomère de celui-ci, un mélange obtenu selon le procédé d'isomères est séparé et l'isomère souhaité est isolé et/ou un composé libre de formule IV selon le procédé ou obtenu d'une autre manière, ou un tautomère de celui-ci, est converti en un sel ou un sel obtenu selon le procédé ou d'une autre manière d'un composé de formule IV ou d'un tautomère de celui-ci, est transformé en le composé libre de formule IV ou en un tautomère de celui-ci, ou en un autre sel.

24. Utilisation d'un composé de formule : 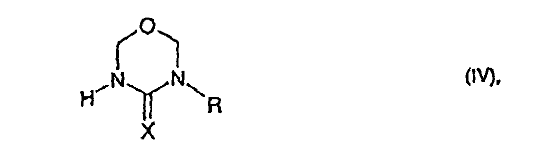 où R et X ont les significations indiquées pour la formule I à la revendication 1, ou un tautomère de celui-ci, chaque fois sous forme libre ou de sel, pour la préparation d'un composé selon la revendication 1 ou d'un tautomère de celui-ci.
